# EUROPÄISCHE PATENTSCHRIFT

(11) **EP 1 309 545 B1**
(45) Veröffentlichungstag und Bekanntmachung des Hinweises auf die Patenterteilung: **22.08.2007**
(21) Anmeldenummer: 01967262.5
(22) Anmeldetag: 10.08.2001
(51) Int. Cl.: C07C 303/06, C11D 1/22, C11D 11/04

(54) **VERFAHREN ZUR HERSTELLUNG VON ALKYLARYLSULFONATEN**
METHOD FOR PRODUCING ALKYL ARYL SULPHONATES
PROCEDE DE PRODUCTION DE SULFONATES D'ALKYLARYLE

(30) Priorität: 11.08.2000 DE 10039995
(43) Veröffentlichungstag der Anmeldung: 14.05.2003
(73) Patentinhaber: BASF Aktiengesellschaft, 67056 Ludwigshafen (DE)
(72) Erfinder: MAAS, Heiko, 68165 Mannheim (DE); NARBESHUBER, Thomas, 68165 Mannheim (DE); RÖPER, Michael, 67157 Wachenheim (DE)
(74) Vertreter: Isenbruck, Günter
(86) Internationale Anmeldenummer: PCT/EP2001/009297
(87) Internationale Veröffentlichungsnummer: WO 2002/014266

(56) Entgegenhaltungen:
- WO-A-88/07030
- WO-A-99/05241
- WO-A-99/07656
- US-A- 3 442 964
- US-A- 5 026 933

## Beschreibung

Die vorliegende Erfindung betrifft Verfahren zur Herstellung von Alkylarylsulfonaten, nach dem Verfahren erhältliche Alkylarylsulfonate sowie in dem Verfahren als Zwischenprodukt erhältliche Alkylaryle, die Verwendung der Alkylarylsulfonate als Tenside, vorzugsweise in Wasch- und Reinigungsmitteln, und diese enthaltende Wasch- und Reinigungsmittel.

Alkylbenzolsulfonate (ABS) werden seit langer Zeit als Tenside in Wasch- und Reinigungsmitteln eingesetzt. Nachdem zunächst derartige Tenside auf Basis von Tetrapropylen eingesetzt wurden, die jedoch schlecht biologisch abbaubar waren, wurden in der Folgezeit möglichst lineare Alkylbenzolsulfonate (LAS) hergestellt und verwendet. Lineare Alkylbenzolsulfonate weisen jedoch nicht in allen Anwendungsbereichen ausreichende Eigenschaftsprofile auf.

So wäre es zum Beispiel vorteilhaft, ihre Kaltwascheigenschaften oder ihre Eigenschaften in hartem Wasser zu verbessern. Ebenso wünschenswert ist die leichte Formulierbarkeit, die sich aus der Viskosität der Sulfonate und deren Löslichkeit ergibt. Diese verbesserten Eigenschaften werden durch geringfügig verzweigte Verbindungen bzw. Mischungen von geringfügig verzweigten Verbindungen mit linearen Verbindungen erreicht, wobei man jedoch das richtige Maß an Verzweigung und/oder das richtige Maß an Mischung erzielen muß. Zu starke Verzweigungen benachteiligen die biologische Abbaubarkeit der Produkte. Zu lineare Produkte beeinflussen die Viskosität und die Löslichkeit der Sulfonate negativ.

Darüber hinaus spielt der Anteil an terminalen Phenylalkanen (2-Phenylalkane und 3-Phenylalkane) zu internen Phenylalkanen (4-, 5-, 6- etc. Phenylalkane) eine Rolle für die Produkteigenschaften. Ein 2-Phenylanteil von etwa 30 % und ein 2- und 3-Phenylanteil von etwa 50 % können hinsichtlich der-Produktqualität (Löslichkeit, Viskosität, Wascheigenschaften) vorteilhaft sein.

Tenside mit sehr hohen 2- und 3-Phenylgehalten können den wesentlichen Nachteil aufweisen, daß die Verarbeitbarkeit der Produkte durch einen starken Anstieg der Viskosität der Sulfonate leidet.

Darüber hinaus kann sich ein nicht-optimales Löslichkeitsverhalten ergeben. So ist z.B. der Krafft-Punkt einer Lösung von LAS mit sehr hohen oder sehr niedrigen 2- und 3-Phenylanteilen um bis zu 10-20 °C höher als bei optimaler Wahl des 2- und 3-Phenylanteils.

Das erfindungsgemäße Verfahren bietet den wesentlichen Vorteil, daß durch die Kombination von Metathese und Dimerisierung ein einzigartiges Olefingemisch erhalten wird, welches nach Alkylierung eines Aromaten, Sulfonierung und Neutralisation ein Tensid liefert, das sich durch seine Kombination von hervorragenden Anwendungseigenschaften (Löslichkeit, Viskosität, Stabilität gegen Wasserhärte, Wascheigenschaften, biologischer Abbaubarkeit) auszeichnet. Hinsichtlich der biologischen Abbaubarkeit von Alkylarylsulfonaten sind Verbindungen, die weniger stark an Klärschlamm adsorbiert werden als herkömmliches LAS, besonders vorteilhaft.

Daher sind zu einem gewissen Grad verzweigte Alkylbenzolsulfonate entwickelt worden.

In der US 3,442,964 ist beispielsweise die Dimerisierung von C₅₋₈-Kohlenwasserstoffen in Gegenwart eines mit einem Übergangsmetall belegten Crackkatalysators beschrieben, wobei vorwiegend zwei- und mehrfach verzweigte Olefine erhalten werden. Diese werden nachfolgend mit Benzol zu einem nicht linearen Alkylbenzol alkyliert. Beispielsweise wird ein Gemisch von Hexenen an einem Siliziumdioxid-Aluminiumoxid-Crackkatalysator dimerisiert und sodann mit Hilfe von HF als Katalysator alkyliert.

WO 88/07030 betrifft Olefine, Alkylbenzole und Alkylbenzolsulfonate, die in Wasch- und Reinigungsmitteln eingesetzt werden können. Dabei wird Propen zu Hexen dimerisiert, das wiederum zu weitgehend linearen Dodecen-Isomeren dimerisiert wird. Sodann wird in Gegenwart von Aluminiumhalogeniden und Flußsäure Benzol alkyliert.

US 5,026,933 beschreibt die Dimerisierung von Propen oder Buten zu Monoolefinen, wobei mindestens 20 % C₁₂-Olefine darstellen, die einen Verzweigungsgrad von 0,8 bis 2,0 Methylgruppen/Alkylkette aufweisen und nur Methylgruppen als Verzweigung aufweisen. Über einem formselektiven Katalysator, vorzugsweise dealuminiertem MOR, werden aromatische Kohlenwasserstoffe alkyliert.

WO 99/05241 betrifft Reinigungsmittel, die verzweigte Alkylarylsulfonate als Tenside enthalten. Die Alkylarylsulfonate werden durch Dimerisierung von Olefinen zu Vinylidinolefinen und nachfolgende Alkylierung von Benzol an einem formselektiven Katalysator wie MOR oder BEA erhalten. Darauf folgt eine Sulfonierung.

Die bislang zur Alkylierung eingesetzten Olefine weisen teilweise einen zu hohen oder zu niedrigen Verweigungsgrad auf bzw. ergeben ein nicht optimales Verhältnis terminaler zu interner Phenylalkane. Zum anderen Teil werden sie aus teuren Ausgangsstoffen wie zum Beispiel Propen oder alpha-Olefinen hergestellt, und teilweise beträgt der Anteil der für die Tensidherstellung interessanten Olefinfraktionen nur etwa 20 %. Dies führt zu teuren Aufarbeitungsschritten.

Aufgabe der vorliegenden Erfindung ist die Bereitstellung eines Verfahrens zur Herstellung von Alkylarylsulfonaten, die zumindest teilweise verzweigt sind und damit für den Einsatz in Wasch- und Reinigungsmitteln gegenüber den bekannten Verbindungen vorteilhafte Eigenschaften aufweisen. Sie sollen insbesondere ein geeignetes Eigenschaftsprofil aus biologischer Abbaubarkeit, Unempfindlichkeit gegen Wasserhärte, Löslichkeit und Viskosität bei der Herstellung und beim Einsatz aufweisen. Zudem sollen die Alkylarylsulfonate kostengünstig herstellbar sein.

Die Aufgabe wird erfindungsgemäß gelöst durch ein Verfahren zur Herstellung von Alkylarylsulfonaten durch
a) Umsetzung eines C₄-Olefin-Gemisches an einem Metathesekatalysator zur Herstellung einer 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens an einem Dimerisierungskatalysator zu einem C₁₀₋₁₂-Olefine enthaltenden Gemisch und gegebenenfalls Abtrennung der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen,
d) Sulfonierung der in Stufe c) erhaltenen alkylaromatischen Verbindungen und Neutralisation zu Alkylarylsulfonaten.

Die Kombination einer Metathese von C₄-Olefinen mit einer nachfolgenden Dimerisierung und Alkylierung von aromatischen Kohlenwasserstoffen erlaubt die Verwendung preisgünstiger Ausgangsstoffe und von Herstellungsverfahren, welche die gewünschten Produkte in hohen Ausbeuten zugänglich machen.

Es wurde erfindungsgemäß gefunden, daß durch Metathese von C₄-Olefinen Produkte erhalten werden, die sich zu leicht verzweigten C₁₀₋₁₂-Olefin-Gemischen dimerisieren lassen. Diese Gemische lassen sich vorteilhaft bei der Alkylierung von aromatischen Kohlenwasserstoffen einsetzen, wobei Produkte erhalten werden, die nach Sulfonierung und Neutralisation Tenside ergeben, die überragende Eigenschaften, insbesondere hinsichtlich der Empfindlichkeit gegen Härte bildenden Ionen, der Löslichkeit der Sulfonate, der Viskosität der Sulfonate und ihrer Wascheigenschaften aufweisen. Darüber hinaus ist das vorliegende Verfahren äußerst kostengünstig, da die Produktströme so flexibel gestaltet werden können, daß keine Nebenprodukte anfallen. Ausgehend von einem C₄-Strom werden durch die erfindungsgemäße Metathese lineare, interne Olefine hergestellt, die sodann über den Dimerisierungsschritt in verzweigte Olefine überführt werden.

Stufe a) des erfindungsgemäßen Verfahrens ist die Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen. Die Metathese kann beispielsweise wie in WO 00/39058 oder DE-A-100 13 253 beschrieben, durchgeführt werden.

Die Olefinmetathese (Disproportionierung) beschreibt in ihrer einfachsten Form die reversible, metallkatalysierte Umalkylidenierung von Olefinen durch Bruch oder Neuformierung von C=C-Doppelbindungen gemäß nachfolgender Gleichung:

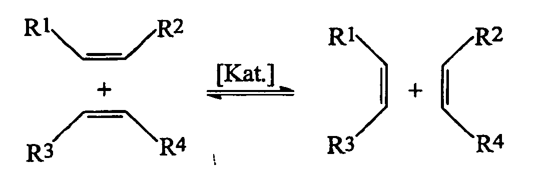

Im speziellen Fall der Metathese von acyclischen Olefinen unterscheidet man zwischen Selbstmetathese, bei der ein Olefin in ein Gemisch zweier Olefine unterschiedlicher molarer Masse übergeht (beispielsweise: Propen → Ethen + 2-Buten), und Kreuz- oder Co-Metathese, die eine Reaktion zweier unterschiedlicher Olefine beschreibt (Propen + 1-Buten → Ethen + 2-Penten). Ist einer der Reaktionspartner Ethen, so spricht man im allgemeinen von einer Ethenolyse.

Als Metathesekatalysatoren eignen sich prinzipiell homogene und heterogene Übergangsmetall-Verbindungen, insbesondere die der VI. bis VIII.-Nebengruppe des Periodensystems der Elemente sowie homogene und heterogene Katalysatorsysteme, in denen diese Verbindungen enthalten sind.

Unterschiedliche Metathese-Verfahren, die von C₄-Strömen ausgehen, sind erfindungsgemäß einsetzbar.

Die DE-A-199 32 060 betrifft ein Verfahren zur Herstellung von C₅-/C₆-Olefinen durch Umsetzung eines Ausgangsstroms, der 1-Buten, 2-Buten und Isobuten enthält, zu einem Gemisch aus C₂₋₆-Olefinen. Dabei wird aus Butenen insbesondere Propen gewonnen. Zusätzlich werden Hexen und Methylpenten als Produkte ausgeschleust. In der Metathese wird kein Ethen zudosiert. Gegebenenfalls wird in der Metathese gebildetes Ethen in den Reaktor zurückgeführt.

Das bevorzugte Verfahren zur Herstellung von gegebenenfalls Propen und Hexen aus einem, olefinische C₄-Kohlenwasserstoffe enthaltenden Raffinat-II-Ausgangsstrom ist dadurch gekennzeichnet, daß
a) in Gegenwart eines Metathesekatalysators, der mindestens eine Verbindung eines Metalls der VI.b, VII.b oder VIII. Nebengruppe des Periodensystems der Elemente enthält, eine Metathesereaktion durchgeführt wird, im Rahmen derer im Ausgangsstrom enthaltene Butene mit Ethen zu einem Ethen, Propen, Butene, 2-Penten, 3-Hexen und Butane enthaltenden Gemisch umgesetzt werden, wobei bezogen auf die Butene bis 0,6 Moläquivalente Ethen eingesetzt werden können,
b) der so erhaltene Austragsstrom zunächst destillativ getrennt wird in gegebenenfalls eine C₂-C₃-Olefine enthaltende Leichtsiederfraktion A sowie in eine C₄-C₆-Olefine und Butane enthaltende Schwersiederfraktion,
c) die aus b) gegebenenfalls erhaltene Leichtsiederfraktion A anschließend destillativ in eine Ethen enthaltende Fraktion und eine Propen enthaltende Fraktion getrennt wird, wobei die Ethen enthaltende Fraktion in den Verfahrensschritt a) zurückgeführt wird und die Propen enthaltende Fraktion als Produkt ausgeschleust wird,
d) die aus b) erhaltene Schwersiederfraktion anschließend destillativ in eine Butene und Butane enthaltende Leichtsiederfraktion B, eine 2-Penten enthaltende Mittelsiederfraktion C und in eine 3-Hexen enthaltende Schwersiederfraktion D getrennt wird,
e) wobei die Fraktionen B und gegebenenfalls C komplett oder teilweise in den Verfahrensschritt a) zurückgeführt werden und die Fraktion D und gegebenenfalls C als Produkt ausgeschleust werden.

Die einzelnen Ströme und Fraktionen können die genannten Verbindungen enthalten oder aus ihnen bestehen. Im Fall, daß sie aus den Strömen oder Verbindungen bestehen, ist die Gegenwart kleinerer Mengen anderer Kohlenwasserstoffe nicht ausgeschlossen.

Dabei wird in einstufiger Reaktionsführung in einer Metathesereaktion eine aus C₄-Olefinen, vorzugsweise n-Butenen und Butanen bestehende Fraktion gegebenenfalls mit variablen Mengen Ethen an einem homogenen oder vorzugsweise heterogenen Metathesekatalysator zu einem Produktgemisch aus (inerten) Butanen, nicht umgesetztem 1-Buten, 2-Buten sowie den Metatheseprodukten Ethen, Propen, 2-Penten und 3-Hexen umgesetzt. Die gewünschten Produkte 2-Penten und/oder 3-Hexen werden ausgeschleust, und die verbleibenden Produkte und nicht umgesetzten Verbindungen werden in die Metathese ganz oder teilweise zurückgeführt. Vorzugsweise werden sie möglichst vollständig zurückgeführt, wobei nur geringe Mengen ausgeschleust werden, um eine Aufpegelung zu vermeiden. Idealerweise kommt es zu keiner Aufpegelung und alle Verbindungen außer 3-Hexen werden in die Metathese zurückgeführt.

Erfindungsgemäß werden, bezogen auf die Butene im C₄-Feedstrom, bis 0,6, vorzugsweise bis 0,5 Moläquivalente Ethen eingesetzt. Damit werden im Vergleich zum Stand der Technik nur geringe Ethenmengen eingesetzt.

Wenn auf die Zuführung von zusätzlichem Ethen verzichtet wird, werden im Verfahren nur bis zu maximal etwa 1,5 %, bezogen auf die Umsetzungsprodukte, an Ethen gebildet, das zurückgeführt wird, siehe DE-A-199 32 060. Es können auch erfindungsgemäß größere Ethenmengen eingesetzt werden, wobei die eingesetzten Mengen wesentlich geringer sind als in den bekannten Verfahren zur Herstellung von Propen.

Zudem werden erfindungsgemäß maximal mögliche Mengen an im Reaktoraustrag enthaltenen C₄-Produkten und gegebenenfalls C₅-Produkten zurückgeführt. Dies betrifft insbesondere die Rückführung von nicht umgesetztem 1-Buten und 2-Buten sowie gegebenenfalls gebildetem 2-Penten.

Sofern im C₄-Feedstrom noch geringe Mengen an Isobuten enthalten sind, können auch geringe Mengen verzweigter Kohlenwasserstoffe gebildet werden.

Die Menge an möglicherweise zusätzlich gebildeten verzweigten C₅- und C₆-Kohlenwasserstoffen im Metatheseaustrag ist abhängig vom Isobuten-Gehalt im C₄-Feed und wird vorzugsweise möglichst gering (< 3 %) gehalten.

Um das erfindungsgemäße Verfahren in mehreren Variationen näher zu erläutern, wird die im Metathesereaktor stattfindende Umsetzung in drei wichtige Einzelreaktionen unterteilt:

### 1. Kreuzmetathese von 1-Buten mit 2-Buten

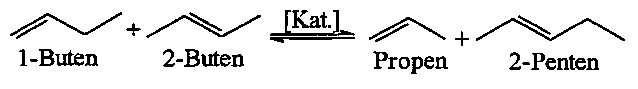

### 2. Selbstmetathese von 1-Buten

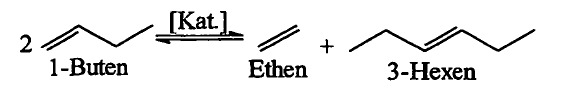

### 3. Gegebenenfalls Ethenolyse von 2-Buten

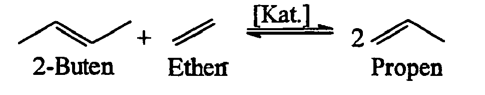

In Abhängigkeit vom jeweiligen Bedarf an den Zielprodukten Propen und 3-Hexen (die Bezeichnung 3-Hexen beinhaltet unter anderem eventuell gebildete Isomere) bzw. 2-Penten kann die äußere Massenbilanz des Verfahrens gezielt durch variablen Einsatz von Ethen und durch Verschiebung des Gleichgewichts durch Rückführung bestimmter Teilströme beeinflußt werden. So wird beispielsweise die 3-Hexenausbeute dadurch erhöht, daß durch Rückführung von 2-Penten in den Metatheseschritt die Kreuzmetathese von 1-Buten mit 2-Buten unterdrückt wird, so daß hier kein oder möglichst wenig 1-Buten verbraucht wird. Bei der dann bevorzugt ablaufenden Selbstmetathese von 1-Buten zu 3-Hexen wird zusätzlich Ethen gebildet, welches in einer Folgereaktion mit 2-Buten zum Wertprodukt Propen reagiert.

Olefingemische, die 1-Buten und 2-Buten und gegebenenfalls Isobuten enthalten, werden u.a. bei diversen Crackprozessen wie Steamcracking oder FCC-Cracking als C₄-Fraktion erhalten. Alternativ können Butengemische, wie sie bei der Dehydrierung von Butanen oder durch Dimerisierung von Ethen anfallen, eingesetzt werden. In der C₄-Fraktion enthaltene Butane verhalten sich inert. Diene, Alkine oder Enine werden vor dem erfindungsgemäßen Metatheseschritt mit gängigen Methoden wie Extraktion oder Selektivhydrierung entfernt.

Der Butengehalt der im Verfahren eingesetzten C₄-Fraktion beträgt 1 bis 100 Gew.-%, vorzugsweise 60 bis 90 Gew.-%. Der Butengehalt bezieht sich dabei auf 1-Buten, 2-Buten und Isobuten.

Vorzugsweise wird eine C₄-Fraktion eingesetzt, wie sie beim Steam- oder FCC-Cracken oder bei der Dehydrierung von Butan anfällt.

Dabei wird als C₄-Fraktion vorzugsweise Raffinat II eingesetzt, wobei der C₄-Strom vor der Metathese-Reaktion durch entsprechende Behandlung an Adsorber-Schutzbetten, bevorzugt an hochoberflächigen Aluminiumoxiden oder Molsieben von störenden Verunreinigungen befreit wird.

Die aus Schritt b) gegebenenfalls erhaltene Leichtsiederfraktion A, die C₂-C₃-Olefine enthält, wird destillativ in eine Ethen enthaltende Fraktion und eine Propen enthaltende Fraktion getrennt. Die Ethen enthaltende Fraktion wird sodann in den Verfahrensschritt a), d.h. die Metathese, zurückgeführt, und die Propen enthaltende Fraktion wird als Produkt ausgeschleust.

In Schritt d) kann die Trennung in Leichtsiederfraktion B, Mittelsiederfraktion C und Schwersiederfraktion D beispielsweise in einer Trennwandkolonne durchgeführt werden. Hierbei wird die Leichtsiederfraktion B über Kopf, die Mittelsiederfraktion C über einen Mittelaustrag und die Schwersiederfraktion D als Sumpf erhalten.

Um die bei dem flexibel gesteuerten Verfahren anfallenden unterschiedlich großen Mengen an Produkten besser handhaben zu können, ist es jedoch vorteilhaft, eine zweistufige Auftrennung der aus b) erhaltenen Schwersiederfraktion durchzuführen. Vorzugsweise wird die aus b) erhaltene Schwersiederfraktion zunächst destillativ in eine Butene und Butane enthaltende Leichtsiederfraktion B und eine 2-Penten und 3-Hexen enthaltende Hochsiederfraktion getrennt. Die Hochsiederfraktion wird sodann destillativ in die Fraktionen C und D getrennt. Die beiden Ausführungsformen sind in den Abbildungen 1 und 2 näher erläutert.

Die Metathesereaktion wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII.-Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxid-Gehalt von 1 bis 20 Gew.-%, vorzugsweise 3 bis 15 Gew.%, besonders bevorzugt 6 bis 12 Gew.-% eingesetzt.

Die Metathese wird bei Flüssigfahrweise vorzugsweise bei einer Temperatur von 0 bis 150°C, besonders bevorzugt 20 bis 80°C sowie einem Druck von 2 bis 200 bar, besonders bevorzugt 5 bis 30 bar, durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 bis 20 bar, besonders bevorzugt 1 bis 5 bar.

Die Herstellung von C₅/C₆-Olefinen und gegebenenfalls Propen aus Steamcracker- oder Raffinerie-C₄-Strömen kann die Teilschritte (1) bis (4) umfassen:
(1) Abtrennung von Butadien und acetylenischen Verbindungen durch gegebenenfalls Extraktion von Butadien mit einem Butadien-selektiven Lösungsmittel und nachfolgend /oder Selektivhydrierung von in Roh-C₄-Schnitt enthaltenen Butadienen und acetylenischen Verunreinigungen um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können, oder Oligomerisierung oder Polymerisation von Isobuten aus dem in der vorstehenden Stufe erhaltenen Reaktionsaustrag in Gegenwart eines sauren Katalysators, dessen Säurestärke zur selektiven Abtrennung von Isobuten als Oligo- oder Polyisobuten geeignet ist, um einen Strom zu erhalten, der 0 bis 15 % Rest-Isobuten aufweist,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinats II-Stromes wie beschrieben.

Vorzugsweise wird der Teilschritt Selek-tivhydrierung von in Roh-C₄-Schnitt enthaltenen Butadien und acetylenischen Verunreinigungen zweistufig durchgeführt durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10, vorzugsweise von 2:1 bis 1:3, vorliegen und im wesentlichen keine Diolefine und acetylenischen Verbindungen enthalten sind. Für einen maximalen Hexenaustrag liegt vorzugsweise 1-Buten im Überschuß vor, für eine hohe Propenausbeute liegt vorzugsweise 2-Buten im Überschuß vor. Das bedeutet, daß das gesamte Molverhältnis im ersten Fall 2:1 bis 1:1 und im zweiten Fall 1:1 bis 1:3 betragen kann.

Vorzugsweise wird der Teilschritt Butadien-Extraktion aus Roh-C₄-Schnitt mit einem Butadien-selektiven Lösungsmittel durchgeführt, ausgewählt aus der Klasse polaraprotischer Lösungsmittel, wie Aceton, Furfural, Acetonitril, Dimethylacetamid, Dimethylformamid und N-Methylpyrrolidon, um einen Reaktionsaustrag zu erhalten, in welchem nach anschließend durchgeführter Selektivhydrierung/Isomerisierung die n-Butene 1-Buten und 2-Buten in einem Molverhältnis 2:1 bis 1:10, vorzugsweise von 2:1 bis 1:3, vorliegen.

Vorzugsweise wird der Teilschritt Isobuten-Veretherung in einer dreistufigen Reaktorkaskade mit Methanol oder Isobutanol, vorzugsweise Isobutanol in Gegenwart eines sauren Ionentauschers durchgeführt, in der geflutete Festbettkatalysatoren von oben nach unten durchströmt werden, wobei die Reaktor-Eingangstemperatur 0 bis 60°C, vorzugweise 10 bis 50°C, die Ausgangstemperatur 25 bis 85°C, vorzugsweise 35 bis 75°C, der Druck 2 bis 50 bar, vorzugsweise 3 bis 20 bar und das Verhältnis von Isobutanol zu Isobuten 0,8 bis 2,0, vorzugsweise 1,0 bis 1,5 beträgt sowie der Gesamtumsatz dem Gleichgewichtsumsatz entspricht.

Vorzugsweise wird der Teilschritt Isobuten-Abtrennung durch Oligomerisierung oder Polymerisation von Isobuten ausgehend von dem nach den vorstehend beschriebenen Stufen Butadien-Extraktion und/oder Selektivhydrierung erhaltenen Reaktionsaustrag in Gegenwart eines Katalysators durchgeführt, der ausgewählt ist aus der Klasse homogener und heterogener Broensted- oder Lewis-Säuren, siehe DE-A-100 13 253.

### Selektivhydrierung von Roh-C₄-Schnitt

Alkine, Alkinene und Alkadiene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark.

Der C₄-Strom eines Steamcrackers enthält einen hohen Anteil mehrfach ungesättigter Verbindungen wie 1,3-Butadien, 1-Butin (Ethylacetylen) und Butenin (Vinylacetylen). Je nach vorhandener Downstream-Verarbeitung werden die mehrfach ungesättigten Verbindungen entweder extrahiert (Butadien-Extraktion) oder selektiv hydriert. Im erstgenannten Fall beträgt der Restgehalt mehrfach ungesättigter Verbindungen typischerweise 0,05 bis 0,3 Gew.-%, im letztgenannten Fall typischerweise 0,1 bis 4,0 Gew.-%. Da die Restmengen an mehrfach ungesättigten Verbindungen ebenfalls bei der Weiterverarbeitung stören, ist eine weitere Anreicherung durch Selektivhydrierung auf Werte < 10 ppm erforderlich. Um einen möglichst hohen Wertproduktanteil an Butenen zu erhalten, ist die Überhydrierung zu Butanen so gering wie möglich zu halten.

### Alternativ: Extraktion von Butadien aus Roh-C₄-Schnitt

Das bevorzugte Verfahren zur Butadien-Isolierung basiert auf dem physikalischen Prinzip der Extraktivdestillation. Durch Zusatz selektiver organischer Lösungsmittel wird die Flüchtigkeit spezieller Komponenten eines Gemisches, in diesem Fall Butadien, erniedrigt. Diese bleiben daher mit dem Lösungsmittel im Sumpf der Destillationskolonne, während die destillativ zuvor nicht abtrennbaren Begleitsubstanzen über Kopf entfernt werden können. Als Lösungsmittel für die Extraktivdestillation kommen hauptsächlich Aceton, Furfural, Acetonitril, Dimethylacetamid, Dimethylformamid (DMF) und N-Methylpyrrolidon (NMP) zur Anwendung. Extraktivdestillationen eignen sich besonders für butadienreiche C₄-Crackschnitte mit einem relativ hohen Anteil an Alkinen, u.a. Methyl-, Ethyl- und Vinylacetylen sowie Methylallen.

Das vereinfachte Prinzip einer Lösungsmittel-Extraktion aus Roh-C₄-Schnitt kann wie folgt dargestellt werden: Der vollständig verdampfte C₄-Schnitt wird einer Extraktionskolonne am unteren Ende zugeführt. Das Lösungsmittel (DMF, NMP) fließt von oben dem Gasgemisch entgegen und belädt sich auf dem Weg nach unten mit besserlöslichem Butadien und geringen Mengen an Butenen. Am unteren Ende der Extraktionskolonne wird ein Teil des gewonnenen Rein-Butadiens zugeführt, um die Butene weitestgehend auszutreiben. Die Butene verlassen die Trennsäule am Kopf. In einer als Ausgaser bezeichneten weiteren Kolonne wird das Butadien durch Auskochen vom Lösungsmittel befreit und anschließend reindestilliert.

Üblicherweise wird der Reaktionsaustrag einer Butadien-Extraktivdestillation in die zweite Stufe einer Selektivhydrierung eingespeist, um den Butadien-Restgehalt auf Werte von < 10 ppm zu reduzieren.

Der nach Abtrennung von Butadien verbleibende C₄-Strom wird als C₄-Raffinat oder Raffinat I bezeichnet und enthält in der Hauptsache die Komponenten Isobuten, 1-Buten, 2-Butene sowie n- und Isobutane.

### Abtrennung von Isobuten aus Raffinat I

Bei der weiteren Auftrennung des C₄-Stromes wird nachfolgend vorzugsweise Isobuten isoliert, da es sich durch seine Verzweigung und seine höhere Reaktivität von den übrigen C₄-Komponenten unterscheidet. Neben der Möglichkeit einer formselektiven Molsiebtrennung, mit welcher Isobuten mit einer Reinheit von 99 % gewonnen werden kann und an den Molsiebporen adsorbierte n-Butene und Butan mittels eines höhersiedenden Kohlenwasserstoffs wieder desorbiert werden können, geschieht dies in erster Linie destillativ unter Verwendung eines sog. Deisobutenizers, mit welchem Isobuten gemeinsam mit 1-Buten und Isobuten über Kopf abgetrennt wird und 2-Butene sowie n-Butan incl. Restmengen an Iso- und 1-Buten im Sumpf verbleiben, oder extraktiv durch Umsetzung von Isobuten mit Alkoholen an sauren Ionenaustauschern. Hierzu werden vorzugsweise Methanol (→ MTBE) oder Isobutanol (IBTBE) eingesetzt.

Die Herstellung von MTBE aus Methanol und Isobuten erfolgt bei 30 bis 100°C und leichtem Überdruck in der Flüssigphase an sauren Ionenaustauschern. Man arbeitet entweder in zwei Reaktoren oder in einem zweistufigen Schachtreaktor, um einen nahezu vollständigen Isobuten-Umsatz (> 99 %) zu erzielen. Die druckabhängige Azeotropbildung zwischen Methanol und MTBE erfordert zur Reindarstellung von MTBE eine mehrstufige Druckdestillation oder wird nach neuerer Technologie durch Methanol-Adsorption an Adsorberharzen erreicht. Alle anderen Komponenten der C₄-Fraktion bleiben unverändert. Da geringe Anteile von Diolefinen und Acetylenen durch Polymerbildung eine Verkürzung der Lebensdauer des Ionenaustauschers bewirken können, werden vorzugsweise bifunktionelle PD-enthaltende Ionenaustauscher eingesetzt, bei denen in Gegenwart kleiner Mengen Wasserstoff nur Diolefine und Acetylene hydriert werden. Die Veretherung des Isobutens bleibt hiervon unbeeinflußt.

MTBE dient in erster Linie zur Octanzahl-Erhöhung von Fahrbenzin. MTBE und IBTBE können alternativ an sauren Oxiden in der Gasphase bei 150 bis 300°C zur Reingewinnung von Isobuten rückgespalten werden.

Eine weitere Möglichkeit zur Abtrennung von Isobuten aus Raffinat I besteht in der direkten Synthese von Oligo/Polyisobuten. An sauren homogenen und heterogen Katalysatoren, wie z.B. Wolframtrioxid auf Titandioxid, kann auf diese Weise bei Isobuten-Umsätzen bis 95 % ein Austragsstrom erhalten werden, der über einen Restanteil an Isobuten von maximal 5 % verfügt.

### Feedreinigung des Raffinat II-Stroms an Adsorbermaterialien

Zur Verbesserung der Standzeit der eingesetzten Katalysatoren für den nachfolgenden Metatheseschritt ist wie vorstehend beschrieben, der Einsatz einer Feed-Reinigung (guard bed) zur Abtrennung von Katalysatorgiften, wie beispielsweise Wasser, Oxygenates, Schwefel oder Schwefelverbindungen bzw. organischen Halogeniden erforderlich.

Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974).

Die Entfernung von speziell Acetaldehyd aus C₃ bis C₁₅-Kohlenwasserstoffen in flüssiger Phase kann gemäß EP-A-0 582 901 erfolgen.

### Selektivhydrierung von Roh-C₄-Schnitt.

Aus der aus einem Steamcracker oder einer Raffinerie stammenden Roh-C₄-Fraktion wird zunächst Butadien (1,2- und 1,3-Butadien) sowie im C₄-Schnitt enthaltene Alkine oder Alkenine in einem zweistufigen Verfahren selektivhydriert. Der aus der Raffinerie stammende C₄-Strom kann gemäß einer Ausführungsform auch direkt in den zweiten Schritt der Selektivhydrierung eingespeist werden.

Der erste Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 40 bis 80°C und einem Druck von 10 bis 30 bar, einem Molverhältnis von Wasserstoff zu Butadien von 10 bis 50 und einer Volumengeschwindigkeit LHSV von bis 15 m³ Frischfeed pro m³ Katalysator pro Stunde und einem Verhältnis von Recycle von Zustrom von 5 bis 20 betrieben.

Der zweite Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich von 50 bis 90°C und einem Druck von 10 bis 30 bar, einem Molverhältnis von Wasserstoff zu Butadien von 1,0 bis 10 und einer Volumengeschwindigkeit LHSV von 5 bis 20 m³ Frischfeed pro m³ Katalysator pro Stunde und einem Verhältnis von Recycle zu Zustrom von 0 bis 15 betrieben.

Der so erhaltene Reaktionsaustrag wird als Raffinat I bezeichnet und weist neben Isobuten 1-Buten und 2-Buten in einem Molverhältnis von 2:1 bis 1:10, vorzugsweise von 2:1 bis 1:3 auf.

### Alternativ: Abtrennung von Butadien aus Roh-C₄-Schnitt via Extraktion

Die Extraktion von Butadien aus Roh-C₄-Schnitt erfolgt unter Verwendung von N-Methylpyrrolidon.
Der Reaktionsaustrag der Extraktion wird gemäß einer Ausführungsform der Erfindung in den zweiten Schritt der vorangehend beschriebenen Selektivhydrierung eingespeist, um Restmengen Butadien zu entfernen, wobei in diesem Selektivhydrierungsschritt das gewünschte Verhältnis 1-Buten zu 2-Buten eingestellt wird.

### Abtrennung von Isobuten via Veretherung mit Alkoholen

In der Veretherungsstufe wird Isobuten mit Alkoholen, vorzugsweise mit Isobutanol, an einem sauren Katalysator, vorzugsweise an einem sauren Ionenaustauscher, zu Ether, vorzugsweise Isobutyl-tert.-butylether umgesetzt. Die Umsetzung erfolgt gemäß einer Ausführungsform der Erfindung in einer dreistufigen Reaktorkaskade, in der geflutete Festbettkatalysatoren von oben nach unten durchströmt werden. Im ersten Reaktor beträgt die Eingangstemperatur 0 bis 60°C, vorzugsweise 10 bis 50°C; die Ausgangstemperatur liegt zwischen 25 und 85°C, vorzugsweise zwischen 35 und 75°C, und der Druck beträgt 2 bis 50 bar, vorzugsweise 3 bis 20 bar. Bei einem Verhältnis von Isobutanol zu Isobuten von 0,8 bis 2,0, vorzugsweise 1,0 bis 1,5 beträgt der Umsatz zwischen 70 und 90 %.

Im zweiten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Der Gesamtumsatz über die zwei Stufen erhöht sich auf 85 bis 99 %, vorzugsweise 90 bis 97 %.

Im dritten und größten Reaktor wird bei gleicher Eingangs- und Ausgangstemperatur von 0 bis 60°C, vorzugsweise 10 bis 50°C, der Gleichgewichtsumsatz erzielt. An die Veretherung und Abtrennung des gebildeten Ethers schließt sich die Etherspaltung an: Die endotherme Reaktion wird an sauren Katalysatoren, vorzugsweise an sauren Heterogenkontakten, beispielsweise Phosphorsäure auf einem SiO₂-Träger, bei einer Eingangstemperatur von 150 bis 300°C, vorzugsweise bei 200 bis 250°C, und einer Ausgangstemperatur von 100 bis 250°C, vorzugsweise bei 130 bis 220°C durchgeführt.

Bei Einsatz von FCC-C₄-Schnitt ist damit zu rechnen, daß Propan in Mengen um 1 Gew.-%, Isobuten in Mengen um 30 bis 40 Gew.-% sowie C₅-Kohlenwasserstoffe in Mengen um 3 bis 10 Gew.-% eingeschleust werden, welche die nachfolgende Verfahrenssequenz beeinträchtigen können. Im Rahmen der Aufarbeitung, des Ethers ist demzufolge die Möglichkeit einer destillativen Abtrennung der genannten Komponenten vorgesehen.

Der so erhaltene, als Raffinat II bezeichnete Reaktionsaustrag weist einen Isobuten-Restgehalt von 0,1 bis 3 Gew.-% auf.

Bei größeren Mengen an Isobuten im Austrag, wie beispielsweise bei Einsatz von FCC-C₄-Fraktionen oder bei der Abtrennung von Isobuten durch sauerkatalysierte Polymerisation zu Polyisobuten (Teilumsatz), kann der verbleibende Raffinatstrom gemäß einer Ausführungsform der Erfindung vor der Weiterverarbeitung destillativ aufbereitet werden.

### Reinigung des Raffinat II-Stroms an Adsorbermaterialien

Der nach der Veretherung/Polymerisation (bzw. Destillation) erhaltene Raffinat II-Strom wird an mindestens einem guard bed, bestehend aus hochoberflächigen Aluminiumoxiden, Kieselgelen, Alumosilikaten oder Molsieben, gereinigt. Das Schutzbett dient hierbei zum Trocknen des C₄-Stroms sowie zur Entfernung von Substanzen, welche als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenaten, organischen Chloriden und Schwefelverbindungen.

Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten.

Als Metathesekatalysatoren werden literaturbekannte heterogene Rhenium-Katalysatoren, wie Re₂O₇ auf γ-Al₂O₃ oder auf Mischträgern, wie z.B. SiO₂/Al₂O₃, B₂O₃/SiO₂/Al₂O₃ oder Fe₂O₃/Al₂O₃ mit unterschiedlichem Metallgehalt bevorzugt. Der Rheniumoxid-Gehalt beträgt unabhängig vom gewählten Träger zwischen 1 und 20 %, vorzugsweise zwischen 3 und 10 %.
Die Katalysatoren werden frisch calziniert eingesetzt und bedürfen keiner weiteren Aktivierung (z.B. durch Alkylierungsmittel). Desaktivierter Katalysator kann durch Abbrennen von Coke-Rückständen bei Temperaturen oberhalb von 400°C im Luftstrom und Abkühlung unter Inertgas-Atmosphäre mehrfach regeneriert werden.

Ein Vergleich der Heterogenkontakte untereinander zeigt, daß Re₂O₇/Al₂O₃ bereits unter sehr milden Reaktionsbedingungen (T = 20 bis 80°C) aktiv ist, während MO₃/SiO₂ (M = Mo, W) erst bei Temperaturen oberhalb von 100 bis 150°C Aktivität entwickelt und demzufolge als Nebenreaktionen C=C-Doppelbindungsisomerisierung auftreten kann.

Ferner sind zu nennen:
- WO₃/SiO₂, präpariert aus (C₅H₅)W(CO)₃Cl und SiO₂ in J. Mol Catal. 1995, 95, 75-83;
- 3-Komponenten-System, bestehend aus [Mo(NO)₂(OR)₂]ₙ, SnEt₄ und AlCl₃ in J. Mol. Catal. 1991, 64, 171-178 und J. Mol. Catal 1989, 57, 207-220;
- Nitridomolybdän (VI)-Komplexe aus hochaktive Präkatalysatoren in J. Organomet. Chem. 1982, 229, C₁₉-C₂₃;
- heterogene SiO₂-geträgerte MoO₃ und WO₃-Katalysatoren in J. Chem. Soc., Faraday Trans. / 1982, 78, 2583-2592;
- geträgerte Mo-Katalysatoren in J. Chem. Soc., Faraday Trans. / 1981, 77, 1763-1777;
- aktive Wolfram-Katalysatorvorstufe in J. Am. Chem. Soc. 1980, 102(21), 6572-6574;
- Acetonitril(pentacarbonyl)wolfram in J. Catal. 1975, 38, 482-484;
- Trichloro(nitrosyl)molybdän(II) als Katalysator-Vorstufe in Z. Chem. 1974, 14, 284-285;
- W(CO)₅PPH₃/EtAlCl₂ in J. Catal. 1974, 34, 196-202;
- WCl₆/n-BuLi in J. Catal 1973, 28, 300-303;
- WCl₆/n-BuLi in J. Catal.1972, 26, 455-458;

FR 2 726 563: O₃ReO[Al(OR)(L)xO]nReO₃ mit R = C₁-C₄₀-Kohlenwasserstoff, n = 1-10, x = 0 oder 1 und L = Solvens,
EP-A-191 0 675, EP-A-129 0 474, BE 899897: Katalysatorsysteme aus Wolfram, 2-substituierten Phenolatresten und 4 anderen Liganden, u.a. einer Halogen-, Alkyl- bzw. Carbengruppe.
FR 2 499 083: Katalysatorsystem aus einem Wolfram-, Molybdän- oder Rhenium-Oxo-Übergangsmetallkomplex mit einer Lewissäure.

US 4,060,468: Katalysatorsystem aus einem Wolframsalz, einer sauerstoffhaltigen aromatischen Verbindung, z.B. 2,6-Dichlorphenol und wahlweise molekularem Sauerstoff.

BE 776,564: Katalysatorsystem aus einem Übergangsmetallsalz, einer metallorganischen Verbindung und einem Amin.

Für die Verbesserung der Cyclusdauer der eingesetzten Katalysatoren, vor allem der geträgerten Katalysatoren, empfiehlt sich der Einsatz einer Feed-Reinigung an Adsorberbetten (guard beds). Das Schutzbett dient hierbei zum Trocknen des C₄-Stroms sowie zur Entfernung von Substanzen, welches als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die bevorzugt so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt. Es kann von Vorteil sein, mehrere Reinigungsschritte miteinander zu kombinieren bzw. hintereinander zu schalten.

Druck und Temperatur im Metatheseschritt sind so gewählt, daß sämtliche Reaktionspartner in der flüssigen Phase vorliegen (üblicherweise = 0 bis 150°C, bevorzugt 20 bis 80°C; p = 2 bis 200 bar). Alternativ kann es aber von Vorteil sein, insbesondere bei Feedströmen mit höherem Isobutengehalt, die Umsetzung in der Gasphase durchzuführen und/oder einen Katalysator einzusetzen, der über eine geringere Acidität verfügt.

In der Regel ist die Umsetzung nach 1 s bis 1 h, vorzugsweise nach 30 s bis 30 min beendet. Sie kann kontinuierlich oder diskontinuierlich in Reaktoren, wie Druckgasgefäßen, Strömungsrohren oder Reaktivdestillationsvorrichtungen durchgeführt werden, wobei Strömungsrohre bevorzugt werden.

### Stufe b)

In Stufe b) wird das in Stufe a) erhaltene 2-Penten und/oder 3-Hexen an einem Dimerisierungskatalysator zu einem C₁₀₋₁₂-Olefin-Gemisch dimerisiert. Gegebenenfalls werden die erhaltenen C₁₀₋₁₂-Olefine abgetrennt.

Bei der Dimerisierung der im Metatheseschritt erhaltenen Olefine oder Olefingemische erhält man Dimerisierungsprodukte, die im Hinblick auf die weitere Verarbeitung zu Alkylaromaten besonders günstige Komponenten und eine besonders vorteilhafte Zusammensetzungen aufweisen, wenn
man einen Dimerisierungskatalysator einsetzt, der wenigstens ein Element der VIII. Nebengruppe des periodischen Systems enthält,
und man die Katalysatorzusammensetzung und die Reaktionsbedingungen so wählt, daß ein Dimerengemisch erhalten wird, welches weniger als 10 Gew.-% von Verbindungen enthält, die ein Strukturelement der Formel I (Vinylidengruppe) 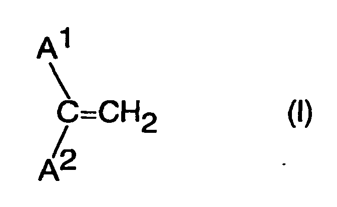 worin A¹ und A² aliphatische Kohlenwasserstoffreste sind,
aufweisen.

Vorzugsweise werden für die Dimerisierung die in dem Metathesierungsprodukt enthaltenen internen, linearen Pentene und Hexene eingesetzt. Besonders bevorzugt ist der Einsatz von 3-Hexen.

Die Dimerisierung kann homogenkatalysiert oder heterogenkatalysiert durchgeführt werden. Bevorzugt ist die heterogene Verfahrensweise, da hierbei einerseits die Katalysatorabtrennung vereinfacht und das Verfahren damit wirtschaftlicher ist, zum anderen werden keine umweltschädlichen Abwässer erzeugt, wie sie gewöhnlich bei der Abtrennung gelöster Katalysatoren, zum Beispiel durch Hydrolyse, anfallen. Ein weiterer Vorteil des heterogenen Verfahrens besteht darin, daß das Dimerisierungsprodukt keine Halogene, insbesondere Chlor oder Fluor, enthält. Homogen lösliche Katalysatoren enthalten im allgemeinen halogenidhaltige Liganden, oder sie werden in Kombination mit halogenhaltigen Cokatalysatoren eingesetzt. Aus solchen Katalysatorsystemen kann Halogen in die Dimerisierungsprodukte eingebaut werden, was sowohl die Produktqualität als auch die Weiterverarbeitung erheblich beeinträchtigt.

Zur heterogenen Katalyse werden zweckmäßigerweise Kombinationen von Oxiden von Metallen der VIII. Nebengruppe mit Aluminiumoxid auf Trägermaterialien aus Silizium-und Titanoxiden wie sie beispielsweise aus der DE-A-43 39 713 bekannt sind, eingesetzt. Der heterogene Katalysator kann im Festbett - dann vorzugsweise in grobkörniger Form als 1 bis 1,5 mm-Splitt - oder suspendiert (Partikelgröße 0.05 bis 0,5 mm) eingesetzt werden. Die Dimerisierung wird bei heterogener Durchführung zweckmäßigerweise bei Temperaturen von 80 bis 200°C, vorzugsweise von 100 bis 180°C, unter dem bei der Reaktionstemperatur herrschenden Druck, gegebenenfalls auch unter einem Schutzgas-überdruck, im geschlossenen System ausgeführt. Zur Erzielung optimaler Umsätze wird das Reaktionsgemisch mehrfach im Kreis geführt, wobei kontinuierlich ein bestimmter Anteil des zirkulierenden Produkts ausgeschleust und durch Ausgangsmaterial ersetzt wird.

Bei der erfindungsgemäßen Dimerisierung werden Mischungen einfach ungesättigter Kohlenwasserstoffe erhalten, deren Komponenten überwiegend die doppelte Kettenlänge haben wie die Ausgangs-Olefine.

Die Dimerisierungskatalysatoren und die Reaktionsbedingungen werden im Rahmen der obigen Angaben vorzugsweise so gewählt, daß mindestens 80 % der Komponenten des Dimerisierungsgemisches im Bereich von ¼ bis 3/4, vorzugsweise von 1/3 bis 2/3, der Kettenlänge ihrer Hauptkette eine Verzweigung, oder zwei Verzweigungen an benachbarten C-Atomen, aufweisen.

Sehr charakteristisch für die erfindungsgemäß hergestellten Olefingemische ist ihr hoher Anteil - in der Regel über 75%, insbesondere über 80% - von Komponenten mit Verzweigungen und der geringe Anteil - in der Regel unter 25, insbesondere unter 20% - unverzweigter Olefine. Ein weiteres Charakteristikum ist, daß an den Verzweigungsstellen der Hauptkette überwiegend Gruppen mit (y-4) und (y-5) C-Atomen gebunden sind, wobei y die Kohlenstoffatom-Anzahl des für die Dimerisierung eingesetzten Monomers ist. Der Wert (y-5)=0 bedeutet, daß keine Seitenkette vorhanden ist.

Bei erfindungsgemäß hergestellten C₁₂-Olefingemischen trägt die Hauptkette an den Verzweigungspunkten vorzugsweise Methyl- oder Ethylgruppen.

Die Stellung der Methyl- und Ethylgruppen an der Hauptkette ist ebenfalls charakteristisch: Bei Monosubstitution befinden sich die Methyl- oder Ethylgruppen in der Position P = (n/2)-m der Hauptkette, wobei n die Länge der Hauptkette und m die Kohlenstoffanzahl der Seitengruppen ist, bei Disubstitutionsprodukten befindet sich ein Substituent in der Position P, der andere am benachbarten C-Atom P+1. Die Anteile von Monosubstitutionsprodukten (Einfachverzweigung) am erfindungsgemäß hergestellten Olefingemisch liegen charakteristischerweise insgesamt im Bereich von 40 bis 75 Gew.-%, die Anteile an doppeltverzweigten Komponenten im Bereich von 5 bis 25 Gew.-%.

Die nach dem vorstehenden Verfahren (vgl. WO 00/39058) erhältlichen Olefingemische stellen wertvolle Zwischenprodukte insbesondere für die im Folgenden beschriebene Herstellung von verzweigten Alkylaromaten zur Herstellung von Tensiden dar.

### Stufe c)

In Stufe c) wird das in Stufe b) erhaltene C₁₀₋₁₂-Olefin-Gemisch mit einem aromatischen Kohlenwasserstoff in Gegenwart eines Alkylierungskatalysators unter Bildung von alkylaromatischen Verbindungen umgesetzt.

Dabei wird vorzugsweise ein Akylierungskatalysator eingesetzt, der zu alkylaromatischen Verbindungen führt, die im Alkylrest ein bis drei Kohlenstoffatome mit einem H/C-Index von 1 aufweisen.

Die Alkylierung kann im Prinzip in Gegenwart beliebiger Alkylierungskatalysatoren durchgeführt werden.

Obwohl AlCl₃ und HF im Prinzip einsetzbar sind, bieten heterogene bzw. formselektive Katalysatoren Vorteile. Aus Gründen der Anlagensicherheit und des Umweltschutzes werden heute Feststoffkatalysatoren bevorzugt, dazu zählen zum Beispiel der im DETAL-Prozeß eingesetzte fluorierte Si/Al-Katalysator, eine Reihe von formselektiven Katalysatoren bzw. geträgerte Metalloxidkatalysatoren, sowie Schichtsilikate und Tone.

Bei der Auswahl des Katalysators ist ungeachtet des großen Einflusses des eingesetzten Feedstocks die Minimierung von durch den Katalysator gebildeten Verbindungen wichtig, die dadurch gekennzeichnet sind, daß sie im Alkylrest C-Atome mit einem H/C-Index von 0 beinhalten. Des weiteren sollen Verbindungen gebildet werden, die im Mittel im Alkylrest 1 bis 3 C-Atome mit einem H/C-Index von 1 aufweisen. Dies kann insbesondere durch die Auswahl geeigneter - Katalysatoren erreicht werden, die einerseits durch ihre Geometrie die Bildung der unerwünschten Produkte unterdrücken und andererseits aber eine ausreichende Reaktionsgeschwindigkeit zulassen.
Der H/C-Index definiert die Anzahl der Protonen pro Kohlenstoffatom im Alkylrest.

Bei der Auswahl der Katalysatoren ist darüber hinaus auf deren Neigung hinsichtlich. Deaktivierung zu achten. Eindimensionale Porensysteme weisen meistens den Nachteil einer raschen Verstopfung der Poren durch Abbau- bzw. Aufbauprodukte aus dem Prozeß auf. Katalysatoren mit mehrdimensionalen Porensystemen sind daher zu bevorzugen.

Die eingesetzten Katalysatoren können natürlichen oder synthetischen Ursprungs sein, deren Eigenschaften sind durch literaturbekannte Methoden (z.B. Ionenaustausch, Steaming, Blockierung azider Zentren, Auswaschen von Extra-Gitter-Spezies, etc.) in gewissem Umfang einstellbar. Wichtig für die vorliegende Erfindung ist, daß die Katalysatoren zumindest zum Teil sauren Charakter aufweisen.

Je nach Anwendungsart liegen die Katalysatoren entweder als Pulver oder als Formkörper vor. Die Verbindungen der Matrizes der Formkörper gewährleisten ausreichende mechanische Stabilität jedoch ist ein freier Zugang der Moleküle zu den aktiven Bestandteilen der Katalysatoren durch ausreichende Porosität der Matrices zu gewährleisten. Die Herstellung solcher Formkörper ist literaturbekannt und wird nach dem Stand der Technik ausgeführt.

### Mögliche Katalysatoren für die Alkylierung (nicht ausschließliche Nennung) sind:

AlCl₃, AlCl₃/Träger (WO 96/26787), HF, H₂SO₄, Ionic Liquids (z.B. WO 98/50153), Perfluorierte Ionenaustauscher bzw. NAFION/Silica (z.B. WO 99/06145), F-Si/Al (US 5,344,997)
Beta (z.B. WO 98/09929, US 5,877,370, US 4,301,316, US 4,301,317) Faujasit (CN 1169889), Schichtsilikate, Tone (EP 711600), Fluorierter Mordenit (WO 00/23405), Mordenit (EP 466558), ZSM-12, ZSM-20, ZSM-38, Mazzite, Zeolith L, Cancrinit, Gmellinit, Offretit, MCM-22, etc.
Bevorzugt sind formselektive 12-Ring Zeolithe.

### Bevorzugte Reaktionsdurchführung

Die Alkylierung wird derart durchgeführt, daß man den Aromaten (das Aromatengemisch) und das Olefin(gemisch) in einer geeigneten Reaktionszone durch Inkontaktbringen mit dem Katalysator reagieren läßt, nach der Reaktion das Reaktionsgemisch aufarbeitet und so die Wertprodukte gewinnt.

Geeignete Reaktionszonen stellen z.B. Rohrreaktoren oder Rührkessel dar. Liegt der Katalysator in fester Form vor, so kann er entweder als Aufschlämmung (Slurry), als Festbett oder als Wirbelbett eingesetzt werden.

Die Reaktionspartner können bei Verwendung eines Festbettreaktors entweder im Gleichstrom oder im Gegenstrom geführt werden. Auch die Ausführung als katalytische Destillation ist möglich.

Die Reaktionspartner liegen entweder in flüssigem und/oder in gasförmigem Zustand vor.

Die Reaktionstemperatur wird so gewählt, daß auf der einen Seite möglichst vollständiger Umsatz des Olefins stattfindet und auf der anderen Seite möglichst wenig Nebenprodukte entstehen. Die Wahl der Temperaturführung hängt außerdem entscheidend vom gewählten Katalysator ab. Reaktionstemperaturen zwischen 50°C und 500°C (bevorzugt 80 bis 350°C, besonders bevorzugt 80-250°C) sind anwendbar.

Der Druck der Reaktion richtet sich nach der gewählten Fahrweise (Reaktortyp) und beträgt zwischen 0,1 und 100 bar, die Katalysatorbelastung (WHSV) wird zwischen 0,1 und 100 gewählt.

Die Reaktionspartner können optional mit inerten Stoffen verdünnt werden. Inerte Stoffe sind bevorzugt Paraffine.

Das Verhältnis von Aromat:Olefin wird üblicherweise zwischen 1:1 und 100:1 (bevorzugt 2:1-20:1) eingestellt.

### Aromatische Einsatzstoffe

Möglich sind alle aromatischen Kohlenwasserstoffe der Formel Ar-R, wobei Ar einen monocyclischen oder bicyclischen aromatischen Kohlenwasserstoff-Rest darstellt und R aus H, C₁₋₅ bevorzugt C₁₋₃-Alkyl, OH, OR etc., bevorzugt H oder C₁₋₃-Alkyl ausgewählt ist. Bevorzugt sind Benzol und Toluol.

### Stufe d)

In Stufe d) werden die in Stufe c) erhaltenen alkylaromatischen Verbindungen sulfoniert und zu Alkylarylsulfonaten neutralisiert.

Die Alkylaryle werden durch
1) Sulfonierung (z.B. mit SO₃, Oleum, Chlorsulfonsäure, etc., bevorzugt mit SO₃ und
2) Neutralisation (z.B. mit Na-, K-, NH₄-, Mg-Verbindungen, bevorzugt mit Na-Verbindungen)
zu Alkylarylsulfonaten umgesetzt. Sulfonierung und Neutralisation sind in der Literatur hinreichend beschrieben und werden nach dem Stand der Technik ausgeführt. Die Sulfonierung wird bevorzugt in einem Fallfilmreaktor ausgeführt, kann aber auch in einem Rührkessel erfolgen. Die Sulfonierung mit SO₃ ist der Sulfonierung mit Oleum vorzuziehen.

Einen beispielhaften Überblick über Alkylierung, Sulfonierung, Neutralistion gibt z.B. "Alkylarylsulfonates: History, Manufacture, Analysis and Environmental Properties" in Surf. Sci. Ser. 56 (1996) Kapitel 2, Marcel Dekker, New York und darin enthaltene Referenzen.

Die Erfindung betrifft auch Alkylarylsulfonate, die nach einem wie vorstehend beschriebenen Verfahren erhältlich sind.

Die erfindungsgemäßen Alkylarylsulfonate werden vorzugsweise als Tenside, insbesondere in Wasch- und Reinigungsmitteln eingesetzt. Die Erfindung betrifft auch ein Wasch-und Reinigungsmittel, enthaltend neben üblichen Inhaltsstoffen Alkylarylsulfonate, wie sie vorstehend beschrieben sind.

Nicht ausschließliche Beispiele üblicher Inhaltsstoffe der erfindungsgemäßen Wasch- und Reinigungsmittel sind im folgenden aufgeführt.

### Bleichmittel

Beispiele sind Alkaliperborate oder Alkalicarbonat-Perhydrate, insbesondere die Natriumsalze.

Ein Beispiel einer verwendbaren organischen Persäure ist Peressigsäure, die vorzugsweise bei der gewerblichen Textilwäsche oder der gewerblichen Reinigung verwendet wird.

Vorteilhaft verwendbare Bleich- oder Textilwaschmittelzusammensetzungen enthalten C₁-₁₂-Percarbonsäuren, C₈₋₁₆-Dipercarbonsäuren, Imidopercarbonsäuren, oder Aryldipercarbonsäuren. Bevorzugte Beispiele verwendbarer Säuren sind Peressigsäure, lineare oder verzweigte Octan-, Nonan-, Decan- oder Dodecanmonopersäuren, Decan- und Dodecandipersäure, Mono- und Diperphthalsäuren, -isophthalsäuren und - terephthalsäuren, Phthalimidopercapronsäure und Terephthaloyldipercapronsäure. Ebenfalls können polymere Persäuren verwendet werden, beispielsweise solche, die Acrylsäuregrundbausteine enthalten, in denen eine Peroxifunktion vorliegt. Die Percarbonsäuren können als freie Säuren oder als Salze der Säuren, vorzugsweise Alkali-oder Erdalkalimetallsalze, verwendet werden.

### Bleichaktivator

Bleichkatalysatoren sind beispielsweise quaternisierte Imine und Sulfonimine, wie sie beispielsweise beschrieben sind in US 5,360,568, US 5,360,569 und EP-A-0 453 003, wie auch Mangan-Komplexe, wie sie beispielsweise beschrieben sind in WO-A 94/21777. Weitere verwendbare metallhaltige Bleichkatalysatoren sind beschrieben in EP-A-0 458 397, EP-A-0 458 398, EP-A-0 549 272.

Bleichaktivatoren sind beispielsweise Verbindungen der nachstehenden Substanzklassen:

Polyacylierte Zucker oder Zuckerderivate mit C₁₋₁₀-Acylresten, vorzugsweise Acetyl-, Propionyl-, Octanoyl-, Nonanoyl- oder Benzoylresten, besonders bevorzugt Acetylresten, sind als Bleichaktivatoren verwendbar. Als Zucker oder Zuckerderivate sind Mono- oder Disaccaride sowie deren reduzierte oder oxidierte Derivate verwendbar, vorzugsweise Glucose, Mannose, Fructose, Saccharose, Xylose oder Lactose. Besonders geeignete Bleichaktivatoren dieser Substanzklasse sind beispielsweise Pentaacetylglucose, Xylosetetraacetat, 1-Benzoyl-2,3,4,6-tetraacetylglucose und 1-Octanoyl-2,3,4,6-tetraacetylglucose.

Eine weitere verwendbare Substanzklasse sind die Acyloxybenzolsufonsäuren und deren Alkali- und Erdalkalimetallsalze, wobei C₁₋₁₄-Acylreste verwendbar sind. Bevorzugt sind Acetyl-, Propionyl-, Octanoyl-, Nonanoyl- und Benzoylreste, insbesondere Acetylreste und Nonanoylreste. Besonders geeignete Bleichaktivatoren dieser Substanzklasse sind Acetyloxybenzolsulfonsäure. Vorzugsweise werden sie in Form ihrer Natriumsalze eingesetzt.

Weiterhin verwendbar sind O-Acyloximester wie z. B. O-Acetylacetonoxim, O-Benzoylacetonoxim, Bis(propylimino)carbonat, Bis(cyclohexylimino)carbonat. Erfindungsgemäß verwendbare acylierte Oxime sind beispielsweise beschrieben in der EP-A-0 028 432. Erfindungsgemäß verwendbare Oximester sind beispielsweise beschrieben in der EP-A-0 267 046.

Ebenfalls verwendbar sind N-Acylcarprolactame wie beispielsweise N-Acetylcaprolactam, N-Benzoylcaprolactam, N-Octanoylcaprolactam, Carbonylbiscaprolactam.

Weiterhin verwendbar sind
- N-diacylierte und N,N'-tetraacylierte Amine, z. B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonyl-carbonamide, z.B. N-Methyl-N-mesyl-acetamid oder N-Methyl-N-mesyl-benzamid;
- N-acylierte cyclische Hydrazide, acylierte Triazole oder Urazole, z.B. Monoacetylmaleinsäurehydrazid;
- O,N,N-trisubstituierte Hydroxylamine, z.B. O-Benzoyl-N,N-succinylhydroxylamin, O-Acetyl-N,N-succinyl-hydroxylamin oder O,N,N-Triacetylhydroxylamin;
- N,N'-diacyl-sulfurylamide, z.B. N,N'-Dimethyl-N,N'-diacetyl-sulfurylamid oder N,N'-Diethyl-N,N'-dipropionyl-sulfurylamid;
- Triacylcyanurate, z.B. Triacetylcyanurat oder Tribenzoylcyanurat;
- Carbonsäureanhydride, z.B. Benzoesäureanhydrid, m-chlorbenzoesäureanhydrid oder Phthalsäureanhydrid;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril;
- diacylierte 2,5-Diketopiperazine, z.B. 1,4-Diacetyl-2,5-diketopiperazin;
- Acylierungsprodukte von Propylendiharnstoff und 2,2-Dimethylpropylendiharnstoff, z.B. Tetraacetylpropylendiharnstoff;
- α-Acyloxy-polyacyl-malonamide, z.B. α-Acetoxy-N,N'-diacetylmalonamid;
- Diacyl-dioxohexahydro-1,3,5-triazine, z.B. 1,5-diacetyl-2,4-dioxohexahydro-1,3,5-triazin.

Ebenso verwendbar sind 1-Alkyl- oder 1-Aryl-(4H)-3,1-benzoxazin-4-one, wie sie beispielsweise beschrieben sind in der EP-B1-0 332 294 und der EP-B 0 502 013. Insbesondere verwendbar sind 2-Phenyl-(4H)-3,1-benzoxazin-4-on und 2-Methyl-(4H)-3,1-benzoxazin-4-on.

Weiterhin verwendbar sind kationische Nitrile wie sie beispielsweise in EP 303 520 und EP 458 391 A1 beschrieben sind. Beispiele für geeignete Kationnitrile sind die Methosulfate oder Tosylate von Trimethylammoniumacetonitril, N,N-Dimethyl-N-octylammonium-acetonitril, 2-(Trimethylammonium)propionitril, 2-(Trimethylammonium)-2-methyl-propionitril, N-Methylpiperazinium-N,N'-diacetonitril und N-Methylmorpholiniumacetronitril.

Besonders geeignete kristalline Bleichaktivatoren sind Tetraacetylethylendiamin (TAED), NOBS, isoNOBS, Carbonylbiscaprolactam, Benzoylcaprolactam, Bis(2-propyl-imino)carbonat, Bis(cyclohexylimino)carbonat, O-Benzoylacetonoxim und 1-Phenyl-(4H)-3,1-benzoxazin-4-on, Anthranil, Phenylanthranil, N-Methylmorpholinoacetonitril, N-Octanoylcaprolactam (OCL) und N-Methylpiperazin-N,N'-diacetonitril sowie flüssige oder schlecht kristallisierende Bleichaktivatoren in einer als Festprodukt konfektionierten Form.

### Bleichstabilisator

Dabei handelt es sich um Additive, die Schwermetallspuren adsorbieren, binden oder komplexieren können. Beispiele für erfindungsgemäß verwendbare Zusätze mit bleichstabilisierender Wirkung sind polyanionische Verbindungen wie Polyphosphate, Polycarboxylate, Polyhydroxypolycarboxylate, lösliche Silikate als vollständig oder teilweise neutralisierte Alkali- oder Erdalkalisalze, insbesondere als neutrale Na- oder Mg-Salze, die relativ schwache Bleichstabilisatoren sind. Starke erfindungsgemäß verwendbare Bleichstabilisatoren sind beispielsweise Komplexbildner, wie Ethylendiamintetraacetat (EDTA), Nitrilotriessigsäure (NTA), Methylglycindiessigsäure (MGDA), β-Alanindiessigsäure (ADA), Ethylendiamin-N,N'-disuccinat (EDDS) und Phosphonate wie Ethylendiamintetramethylenphosphonat, Diethylentriaminpentamethylenphosphonat oder Hydroxyethyliden-1,1-diphosphonsäure in Form der Säuren oder als teilweise oder vollständig neutralisierte Alkalimetallsalze. Vorzugsweise werden die Komplexbildner in Form ihrer Na-Salze eingesetzt.

Vorzugsweise enthalten die erfindungsgemäßen Waschmittel mindestens einen Bleichstabilisator, besonders bevorzugt mindestens einen der o.g. starken Bleichstabilisatoren.

Auf dem Gebiet der Textilwäsche, der Bleiche und der Reinigung im Haushalt und im gewerblichen Bereich können die beschriebenen Bleich- oder Textilwaschmittelzusammensetzungen gemäß einer Ausführungsform der Erfindung nahezu alle üblichen Bestandteilen von Wasch-, Bleich- und Reinigungsmitteln enthalten. Man kann auf diese Weise beispielsweise Mittel aufbauen, die sich speziell zur Textilbehandlung bei niederen Temperaturen eignen, und auch solche, die in mehreren Temperaturbereichen bis hinauf zum traditionellen Bereich der Kochwäsche geeignet sind.

Hauptbestandteile von Textilwasch- und Reinigungsmitteln sind neben Bleichmittelzusammensetzungen Gerüstsubstanzen (Builder), d. h. anorganische Builder und/oder organische Cobuilder, und Tenside, insbesondere anionische und/oder nichtionische Tenside. Daneben können andere übliche Hilfsstoffe und Begleitstoffe wie Stellmittel, Komplexbildner, Phosphonate, Farbstoffe, Korrosionsinhibitoren, Vergrauungsinhibitoren und/oder Soil-Release-Polymere, Farbübertragungsinhibitoren, Bleichkatalysatoren, Peroxidstabilisatoren, Elektrolyte, optische Aufheller, Enzyme, Parfumöle, -Schaumregulatoren und aktivierende Substanzen in diesen Mitteln vorliegen, wenn dies zweckmäßig ist.

### Anorganische Builder (Gerüstsubstanzen)

Als anorganische Buildersubstanzen eignen sich alle üblichen anorganischen Builder wie Alumosilikate, Silikate, Carbonate und Phosphate.

Geeignete anorganische Builder sind z.B. Alumosilikate mit ionenaustauschenden Eigenschaften wie z.B. Zeolithe. Verschiedene Typen von Zeolithen sind geeignet, insbesondere Zeolith A, X, B, P, MAP und HS in ihrer Na-Form oder in Formen, in denen Na teilweise gegen andere Kationen wie Li, K, Ca, Mg oder Ammonium ausgetauscht sind. Geeignete Zeolithe sind beispielsweise beschrieben in EP-A 038 591, EP-A 021 491, EP-A 087 035, US-A 4,604,224, GB-A2 013 259, EP-A 522 726, EP-A 384 070 und WO-A 94/24 251.

Weitere geeignete anorganische Builder sind z.B. amorphe oder kristalline Silikate wie z.B. amorphe Disilikate, kristalline Disilikate wie das Schichtsilikat SKS-6 (Hersteller Hoechst). Die Silikate können in Form ihrer Alkali-, Erdalkali- oder Ammoniumsalze eingesetzt werden. Vorzugsweise werden Na-, Li- und Mg-Silikate eingesetzt.

### Anionische Tenside

Geeignete anionische Tenside sind die erfindungsgemäßen linearen und/oder -leicht verzweigten Alkylbenzolsulfonate (LAS).

Weitere geeignete anionische Tenside sind beispielsweise Fettalkoholsulfate von Fettalkoholen mit 8 bis 22, vorzugsweise 10 bis 18 Kohlenstoffatomen, z.B. C₉- bis C₁₁-Alkoholsulfate, C₁₂- bis C₁₃-Alkoholsulfate, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, daß man zunächst einen C₈- bis C₂₂-, vorzugsweise einen C₁₀- bis C₁₈-Alkohol, z.B. einen Fettalkohol, alkoxyliert und das Alkoxylierungsprodukt anschließend sulfatiert. Für die Alkoxylierung verwendet man vorzugsweise Ethylenoxid, wobei man pro Mol Fettalkohol 2 bis 50, vorzugsweise 3 bis 20 mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid enthalten. Die alkoxylierten C₈- bis C₂₂-Alkohole können die Ethylenoxid-, Propylenoxid- und Butylenoxideinheiten in Form von Blöcken oder in statistischer Verteilung enthalten.

Weitere geeignete anionische Tenside sind N-Acylsarkosinate mit aliphatischen gesättigten oder ungesättigten C₈- bis C₂₅-Acylresten, vorzugsweise C₁₀- bis C₂₀-Acylresten, z. B. N-Oleoylsarkosinat.

Die anionischen Tenside werden dem Waschmittel vorzugsweise in Form von Salzen zugegeben. Geeignete Kationen in diesen Salzen sind Alkalimetallsalze wie Natrium, Kalium und Lithium und Ammoniumsalze wie z.B. Hydroxyethylammonium-, Di(hydroxyethyl)ammonium- und Tri(hydroxyethyl)ammoniumsalze.

Vorzugsweise enthalten die erfindungsgemäßen Waschmittel C₁₀- bis C₁₃-lineare und/oder -leicht verzweigte Alkylbenzolsulfonate (LAS).

### Nichtionische Tenside

Als nichtionische Tenside eignen sich beispielsweise alkoxylierte C₈- bis C₂₂-Alkohole wie Fettalkoholalkoxylate oder Oxaalkoholalkoxylate. Die Alkoxylierung kann mit Ethylenoxid, Propylenoxid und/oder Butylenoxid durchgeführt werden. Als Tensid einsetzbar sind hierbei sämtliche alkoxylierten Alkohole, die mindestens zwei Moleküle eines vorstehend genannten Alkylenoxids addiert enthalten. Auch hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Pro Mol Alkohol verwendet man 2 bis 50, vorzugsweise 3 bis 20 mol mindestens eines Alkylenoxids. Vorzugsweise setzt man als Alkylenoxid Ethylenoxid ein. Die Alkohole haben vorzugsweise 10 bis 18 Kohlenstoffatome.

Eine weitere Klasse geeigneter nichtionischer Tenside sind Alkylphenolethoxylate mit C₆-bis C₁₄-Alkylketten und 5 bis 30 mol Ethylenoxideinheiten.

Eine andere Klasse nichtionischer Tenside sind Alkylpolyglucoside mit 8 bis 22, vorzugsweise 10 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Struktur II oder m 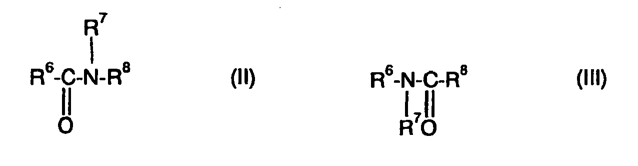 wobei R⁶ C₆- bis C₂₂-Alkyl, R7 H oder C₁- bis C₄-Alkyl und R8 ein Polyhydroxyalkyl-Rest mit 5 bis 12 C-Atomen und mindestens 3 Hydroxygruppen ist. Vorzugsweise ist R₆ C₁₀-bis C₁₈-Alkyl, R⁷ Methyl und R⁸ ein C₅- oder C₆-Rest. Beispielsweise erhält man derartige Verbindungen durch die Acylierung von reduzierend aminierten Zuckern mit Säurechloriden von C₁₀-C₁₈-Carbonsäuren.

Vorzugsweise enthalten die erfindungsgemäßen Waschmittel mit 3 - 12 mol Ethylenoxid ethoxylierte C₁₀-C₁₆-Alkohole, besonders bevorzugt ethoxylierte Fettalkohole als nichtionische Tenside.

### Organischer Cobuilder

Geeignete niedermolekulare Polycarboxylate als organische Cobuilder sind beispielsweise:
C₄- bis C₂₀-Di-, -Tri- und -Tetracarbonsäuren wie z.B. Bernsteinsäure, Propantricarbonsäure, Butantetracarbonsäure, Cyclopentantetracarbonsäure und Alkyl- und Alkenylbernsteinsäuren mit C₂- bis C₁₆-Alkyl- bzw. Alkenyl-Resten;
C₄- bis C₂₀-Hydroxycarbonsäuren wie z.B. Äpfelsäure, Weinsäure, Gluconsäure, Glucarsäure, Citronensäure" Lactobionsäure und Saccharosemono-, -di- und tricarbonsäure;
Aminopolycarboxylate wie z.B. Nitrilotriessigsäure, Methylglycindiessigsäure, Alanindiessigsäure; Ethylendiamintetraessigsäure und Serindiessigsäure;
Salze von Phosphonsäuren wie z.B. Hydroxyethandiphosphonsäure, Ethylendiamintetra(methylenphosphonat) und Diethylentriaminpenta(methylenphosphonat).
Geeignete oligomere oder polymere Polycarboxylate als organische Cobuilder sind beispielsweise:
   Oligomaleinsäuren, wie sie beispielsweise in EP-A-451 508 und EP-A-396 303 beschrieben sind;
   Co- und Terpolymere ungesättigter C₄-C₈-Dicarbonsäuren, wobei als Comonomere monoethylenisch ungesättigte Monomere
   aus der Gruppe (i) in Mengen von bis zu 95 Gew.-%
      aus der Gruppe (ii) in Mengen von bis zu 60 Gew.-%
      aus der Gruppe (iii) in Mengen von bis zu 20 Gew.-%
      einpolymerisiert enthalten sein können.

Als ungesättigte C₄-C₈-Dicarbonsäuren sind hierbei beispielsweise Maleinsäure, Fumarsäure, Itaconsäure und Citraconsäure geeignet Bevorzugt ist Maleinsäure.

Die Gruppe (i) umfaßt monoethylenisch ungesättigte C₃-C₈-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfaßt monoethylenisch ungesättigte C₂-C₂₂-Olefine, Vinylalkylether mit C₁-C₈-Alkylgruppen, Styrol, Vinylester von C₁-C₈ Carbonsäuren, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt.

Die Gruppe (iii) umfaßt (Meth)acrylester von C₁-bisC₈-Alkoholen, (Meth)acrylnitril, (Meth)acrylamide von C₁-C₈-Aminen, N-Vinylformamid und Vinylimidazol.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co-und Terpolymere sind beispielsweise aus US-A 3 887 806 sowie DE-A43 13 909 bekannt.

Als Copolymere von Dicarbonsäuren eignen sich als organische Cobuilder vorzugsweise:

Copolymere von Maleinsäure und Acrylsäure im Gewichtsverhältnis 10:90 bis 95:5, besonders bevorzugt solche im Gewichtsverhältnis 30:70 bis 90:10 mit Molmassen von 10.000 bis 150.000;

Terpolymere aus Maleinsäure, Acrylsäure und einem Vinylester einer C₁-C₃-Carbonsäure im Gewichtsverhältnis 10(Maleinsäure):90(Acrylsäure + Vinylester) bis 95(Maleinsäure):5 (Acrylsäure + Vinylester), wobei das Gew.-Verhältnis von Acrylsäure zu Vinylester im Bereich von 20:80 bis 80:20 variieren kann, und besonders bevorzugt

Terpolymere aus Maleinsäure, Acrylsäure und Vinylacetat oder Vinylpropionat im Gewichtsverhältnis 20(Maleinsäure):80(Acrylsäure + Vinylester) bis 90(Maleinsäure):10(Acrylsäure + Vinylester), wobei das Gewichtsverhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobutan im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5,227,446, DE-A-44 15 623, DE-A-43 13 909, sind ebenfalls als organische Cobuilder geeignet.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z.B saure oder enzymatisch abgebaute Stärken, Inuline oder Zellulose, reduzierte (hydrierte oder hydrierend aminierte) abgebaute Polysaccharide wie z.B. Mannit, Sorbit, Aminosorbit und Glucamin geeignet sowie Polyalkylenglycole mit Molmassen bis zu M_{w} = 5.000 wie z.B Polyethylenglycole, Ethylenoxid/Propylenoxid- bzw Ethylenoxid/Butylenoxid-Blockcopolymere, statistische Ethylenoxid/Propylenoxid- bzw. Ethylenoxid/Butylenoxid-Copolymere, alkoxylierte ein-oder mehrbasische C₁-C₂₂-Alkohole, vgl. US-A4,746,456.

Bevorzugt werden aus dieser Gruppe gepfropfte abgebaute bzw abgebaute reduzierte Stärken und gepfropfte Polyethylenoxide eingesetzt, wobei 20 bis 80 Gew.-% Monomere bezogen auf die Pfropfkomponente bei der Pfropfpolymerisation eingesetzt werden. Zur Pfropfung wird vorzugsweise eine Mischung von Maleinsäure und Acrylsäure im Gew.-Verhältnis von 90:10 bis 10:90 eingesetzt.

Polyglyoxylsäuren als organische Cobuilder sind beispielsweise beschrieben in EP-B-001 004, US-A 5,399,286, DE-A-41 06 355 und EP-A-656 914. Die Endgruppen der Polyglyoxylsäuren können unterschiedliche Strukturen aufweisen.

Polyamidocarbonsäuren und modifizierte Polyamidocarbonsäuren als organische Cobuilder sind beispielsweise bekannt aus EP-A-454 126, EP-B-511 037, WO-A 94/01486 und EP-A-581 452.

Vorzugsweise verwendet man als organische Cobuilder auch Polyasparaginsäure oder Cokondensate der Asparaginsäure mit weitern Aminosäuren, C₄-C₂₅-Mono-oder - Dicarbonsäuren und/oder C₄-C₂₅-Mono- oder -Diaminen. Besonders bevorzugt werden in phosphorhaltigen Säuren hergestellte, mit C₆-C₂₂-Mono- oder -Dicarbonsäuren bzw. mit C₆-C₂₂-Mono- oder -Diaminen modifizierte Polyasparaginsäuren eingesetzt.

Kondensationsprodukte der Citronensäure mit Hydroxycarbonsäuren oder Polyhydroxyverbindungen als organische Cobuilder sind z.B. bekannt aus WO-A 93/22362 und WO-A 92/16493. Solche Carboxylgruppen enthaltende Kondensate haben üblicherweise Molmassen bis zu 10.000, vorzugsweise bis zu 5.000.

### Vergrauungsinhibitoren und Soil-Release-Polymere

Geeignete Soil-Release-Polymere und/oder Vergrauungsinhibitoren für Waschmittel sind beispielsweise:
Polyester aus Polyethylenoxiden mit Ethylenglycol und/oder Propylenglycol und aromatischen Dicarbonsäuren oder aromatischen und aliphatischen Dicarbonsäuren;
Polyester aus einseitig endgruppenverschlossenen Polyethylenoxiden mit zwei-und/oder mehrwertigen Alkoholen und Dicarbonsäure.
Derartige Polyester sind bekannt, beispielsweise aus US-A 3,557,039, GB-A 1 154 730, EP-A-185 427, EP-A-241 984, EP-A-241 985, EP-A- 272 033 und US-A 5,142,020.
Weitere geeignete Soil-Release-Polymere sind amphiphile Pfropf- oder Copolymere von Vinyl-und/oder Acrylestern auf Polyalkylenoxide (vgl. US-A 4,746,456, US-A 4,846,995, DE-A-37 11 299, US-A 4,904,408, US-A 4,846,994 und US-A 4,849,126) oder modifizierte Cellulosen wie z.B. Methylcellulose, Hydroxypropylcellulose oder Carboxymethylcellulose.

### Farbübertragungsinhibitoren

Als Farbübertragungsinhibitoren werden beispielsweise Homo- und Copolymere des Vinylpyrrolidons, des Vinylimidazols, des Vinyloxazolidons und des 4-Vinylpyridm-N-oxids mit Molmassen von 15.000 bis 100.000 sowie vernetzte feinteilige Polymere auf Basis dieser Monomeren eingesetzt. Die hier genannte Verwendung solcher Polymere ist bekannt, vgl. DE-B- 22 32 353, DE-A-28 14 287, DE-A-28 14 329 und DE-A-43 16 023.

### Enzyme

Geeignete Enzyme sind beispielsweise Proteasen, Amylasen, Lipasen und Cellulasen, insbesondere Proteasen. Es können mehrere Enzyme in Kombination verwendet werden.

Neben der Anwendung in Wasch- und Reinigungsmitteln für die Textilwäsche im Haushalt sind die erfindungsgemäß verwendbaren Waschmittelzusammensetzungen auch im Bereich der gewerblichen Textilwäsche und der gewerblichen Reinigung einsetzbar. In der Regel wird in diesem Einsatzbereich Peressigsäure als Bleichmittel eingesetzt, die als wäßrige Lösung der Waschflotte zugesetzt wird.

### Verwendung in Textilwaschmitteln

Ein typisches erfindungsgemäßes pulver- oder granulatförmiges Vollwaschmittel kann beispielsweise folgende Zusammensetzung aufweisen:
- 0,5 bis 50 Gew.-%, vorzugsweise 5 bis 30 Gew.-%, mindestens eines anionischen und/oder nichtionischen Tensids,
- 0,5 bis 60 Gew.-%, vorzugsweise 15 bis 40 Gew.-%, mindestens eines anorganischen Builders,
- 0 bis 20 Gew.-%, vorzugsweise 0,5 bis 8 Gew.-%, mindestens eines organischen Cobuilders,
- 2 bis 35 Gew.-%, vorzugsweise 5 bis 30 Gew.-%, eines anorganischen Bleichmittels,
- 0,1 bis 20 Gew.-%, vorzugsweise 0,5 bis 10 Gew.-%, eines Bleichaktivators, gegebenenfalls in Abmischung mit weiteren Bleichaktivatoren,
- 0 bis 1 Gew.-%, vorzugsweise bis höchstens 0,5 Gew.-%, eines Bleichkatalysators,
- 0 bis 5 Gew.-%, vorzugsweise 0 bis 2,5%, eines polymeren Farbübertragungsinhibitors,
- 0 bis 1,5 Gew.-%, vorzugsweise 0,1 bis 1,0 Gew.-%, Protease,
- 0 bis 1,5 Gew.-%, vorzugsweise 0,1 bis 1,0 Gew.-%, Lipase,
- 0 bis 1,5 Gew.-%, vorzugsweise 0,2 bis 1,0 Gew.-% eines Soil-Release-Polymers, ad 100% übliche Hilfs- und Begleitstoffe und Wasser.

Vorzugsweise in Waschmitteln eingesetzte anorganische Builder sind Natriumcarbonat, Natriumhydrogencarbonat, Zeolith A und P sowie amorphe und kristalline Na-Silikate.

Vorzugsweise in Waschmitteln eingesetzte organische Cobuilder sind Acrylsäure/Maleinsäure-Copolymere, Acrylsäure/Maleinsäure/Vinylester- Terpolymere und Citronensäure.

Vorzugsweise in Waschmitteln eingesetzte anorganische Bleichmittel sind Natriumperborat und Natriumcarbonat-Perhydrat.

Vorzugsweise in Waschmitteln eingesetzte anionische Tenside sind die erfindungsgemäßen linearen und leicht verzweigten Alkylbenzolsulfonate (LAS), Fettalkoholsulfate und Seifen.
Vorzugsweise in Waschmitteln eingesetzte nichtionische Tenside sind C₁₁- bis C₁₇-Oxoalkoholethoxylate mit 3-13 Ethylenoxid-Einheiten, C₁₀- bis C₁₆-Fettalkoholethoxylate mit 3-13 Ethylenoxideinheiten sowie zusätzlich mit 1-4 Propylenoxid- oder ButylenoxidEinheiten alkoxylierte ethoxylierte Fett- oder Oxoalkohole.

Vorzugsweise in Waschmitteln eingesetzte Enzyme sind Protease, Lipase und Cellulase. Von den handelsüblichen Enzymen werden dem Waschmittel in der Regel Mengen von 0,05 bis 2,0 Gew.-%, vorzugsweise 0,2 bis 1,5 Gew.-%, des konfektionierten Enzyms zugesetzt. Geeignete Proteasen sind z.B Savinase, Desazym und Esperase (Hersteller Novo Nordisk). Eine geeignete Lipase ist z.B. Lipolase (Hersteller Novo Nordisk). Eine geeignete Cellulase ist z.B. Celluzym (Hersteller Novo Nordisk).

Vorzugsweise in Waschmitteln eingesetzte Vergrauungsinhibitoren und Soil-Release-Polymere sind Pfropfpolymere von Vinylacetat auf Polyethylenoxid der Molmasse 2.500-8.000 im Gewichtsverhältnis 1,2:1 bis 3,0:1, Polyethylenterephthalate/Oxyethylenterephthalate der Molmasse 3.000 bis 25.000 aus Polyethylenoxiden der Molmasse 750 bis 5.000 mit Terephthalsäure und Ethylenoxid und einem Molverhältnis von Polyethylenterephthalat zu Polyoxyethylenterephthalat von 8:1 bis 1:1 sowie Blockpolykondensate gemäß DE-A-44 03 866.

Vorzugsweise in Waschmitteln eingesetzte Farbübertragungsinhibitoren sind lösliche Vinylpyrrolidon- und Vinylimidazol-Copolymere mit Molmassen über 25.000 sowie feinteilige vernetzte Polymere auf Basis Vinylimidazol.

Die erfindungsgemäßen pulver- oder granulatförmigen Waschmittel können bis zu 60 Gew.-% anorganischer Stellmittel enthalten. Üblicherweise wird hierfür Natriumsulfat verwendet. Vorzugsweise sind die erfindungsgemäßen Waschmittel aber arm an Stellmitteln und enthalten nur bis zu 20 Gew.-%, besonders bevorzugt nur bis 8Gew.-% an Stellmitteln.

Die erfindungsgemäßen Waschmittel können unterschiedliche Schüttdichten im Bereich von 300 bis 1.200, insbesondere 500 bis 950g/1, besitzen. Moderne Kompaktwaschmittel besitzen in der Regel hohe Schüttdichten und zeigen einen Granulataufbau.
Die Erfindung wird anhand der nachstehenden Beispiele näher erläutert.

### Beispiel 1

Eine butadienfreie C₄-Fraktion mit einem Gesamtbutengehalt von 84,2 Gew.-% sowie einem Molverhältnis 1-Buten zu 2-Butene von 1 zu 1,06 wird bei 40°C und 10 bar kontinuierlich über einen mit Re₂O₇/Al₂O₃-Heterogenkontakt bestückten Rohrreaktor geleitet. Die Katalysator-Belastung beträgt im Beispiel 4500 kg/m²h. Der Reaktionsaustrag wird destillativ getrennt und enthält folgende Komponenten (Angaben in Massenprozent):
Ethen 1,15 %; Propen 18,9 %, Butane 15,8 %, 2-Butene 19,7 %, 1-Buten 13,3 %, i-Buten 1,0 %, 2-Penten 19,4 %, Methylbutene 0,45 %, 3-Hexen 10,3 %.
2-Penten und 3-Hexen werden aus dem Produkt destillativ in Reinheiten > 99 Gew.-% gewonnen.

### Beispiel 2

Kontinuierliche Dimerisierung von 3-Hexen im Festbettverfahren

| | |
|---|---|
| Katalysator: | 50 % NiO, 34 % SiO₂, 13 % TiO₂, 3 % Al₂O₃ (gemäß DE 43 39 713) eingesetzt als 1-1,5 mm Splitt (100 ml), 24 h bei 160°C in N₂ konditioniert |
| Reaktor: | isotherm, 16 mm-Ø-Reaktor |
| WHSV: | 0,25 kg/l.h |
| Druck: | 20 bis 25 bar |
| Temperatur: | 100 bis 160°C |

| | | | | | | | | |
|---|---|---|---|---|---|---|---|---|
| Temperatur | | 100 | 120 | 140 | 160 | 160 | | |
| (°C) | | | | | | | | |
| Druck | Feed- | 20 | 20 | 20 | 25 | 25 | Sammel | C₁₂- |
| (bar) | stock | | | | | | - | Destillat |
| Betriebsstunden | | 12 | 19 | 36 | 60 | 107 | Produkt | |
| Flüssiganfall | | 24 | 27 | 27 | 28 | 27 | | |
| (g/h) | | | | | | | | |
| Zusammensetzung | | | | | | | | |
| (Gew.-%) | | | | | | | | |
| C₆ | 99,9 | 68,5 | 52,7 | 43,6 | 57,0 | 73,2 | n.e. | 0,1 |
| C₇ - C₁₁ | 0,1 | 0,2 | 0,2 | 0,3 | 0,2 | 0,2 | | - |
| C₁₂ | | 25,9 | 38,6 | 44,0 | 35,6 | 23,6 | | 99,9 |
| C₁₃+ | | 5,4 | 8,5 | 12,1 | 7,2 | 3,0 | | - |
| | | | | | | | | |
| Umsatz | | 31,4 | 47,2 | 56,4 | 42,9 | 26,7 | | |
| C12-Selektivität | | 82,5 | 81,8 | 78,2 | 83,0 | 88,4 | | |
| (Gew.-%) | | | | | | | | |
| S-Gehalt im | < 1 | n.e. | n.e. | n.e. | n.e. | n.e. | n.e. | n.e. |
| Flüssiganfall | | | | | | | | |
| (ppm) | | | | | | | | |

Das Sammel-Produkt wurde bis zu einer C₁₂-Reinheit von 99,9 Gew.-% aufdestilliert.

### Beispiel 3

2-Penten aus der Raffinat II-Metathese wurde analog zu Beispiel 2 kontinuierlich an einem Ni-Hetrogenkatalysator dimerisiert. Durch fraktionierte Destillation des Produktes wurde eine Decenfraktion mit einer Reinheit von 99,5 % erhalten.

### Beispiel 4

Eine Mischung von 2-Penten und 3-Hexen aus der Raffinat II-Metathese wurde analog zu Beispiel 2 und Beispiel 3 kontinuierlich dimerisiert. Durch fraktionierte Destillation des Produktes wurde eine Decen-/Undecen-/Dodecenfraktion mit einer Reinheit von 99,5 % erhalten.

### Beispiel 5

Die C₁₂-Olefin-Fraktion aus Beispiel 2 wird mit Benzol im molaren Verhältnis 1:10 alkyliert. Dazu wird die Reaktionsmischung in einen Autoklaven (300ml) verbracht, der mit einem Rührer und einem Katalysatorkorb versehen ist. In den Katalysatorkorb werden 25 Gew% bezogen auf die Masse des Olefins an Katalysator Zeolith Mordenith (MOR) gefüllt. Der Autoklav wird verschlossen und zweimal mit Stickstoff (N₂) gespült. Der Autoklav wird dann auf 180°C aufgeheizt. Man läßt die Reaktionsmischung 12h reagieren, kühlt dann ab, filtriert etwaige Katalysatorpartikel aus der Reaktionsmischung und analysiert die Reaktionsmischung mittels Gaschromatographie-Massenspektrometrie Kopplung.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 6

In einen 21 Vierhalskolben mit Magnetrührer, Thermometer, Tropftrichter, Gaseinleitfritte und Gasausgang werden 1900 g an SO₃-verarmtem Oleum vorgelegt. Über den Gasausgang ist dieser Kolben mit einem 1 L Dreihalskolben über einen Vitonschlauch verbunden.
In diesem 1 L Kolben mit Blattrührer, Thermometer, Gaseinleitfritte und Gasausgang wird die Alkylbenzolmischung aus Beispiel 5 vorgelegt.
Das verarmte Oleum wird im SO₃-Entwickler auf 120° C gebracht und das Oleum (65%ig) über einen Tropftrichter innerhalb von 30 Minuten zugegeben. Mit einem Stickstoffstrom von 80 L/h wird das SO₃-Gas ausgestrippt und über ein 6mm Einleitrohr in das Alkylbenzol eingeleitet. Die Temperatur der Alkylbenzol/Alkylbenzolsulfonsäure-Mischung steigt langsam auf 40 °C an und wird mit Kühlwasser auf 40 °C gehalten. Das Restgas wird über eine Wasserstrahlpumpe abgesaugt.
Das molare Verhältnis von SO₃/Alkylbenzol beträgt 1,01:1.
Die gebildete Alkylbenzol-Sulfonsäure wird nach einer Nachreaktionszeit von 4h mit 0,4Gew% Wasser stabilisiert und danach mit NaOH zum Alkylbenzolsulfonat neutralisiert.

### Beispiel 7

Eine Mischung der C₁₀-/ C₁₁/C₁₂-Olefin-Fraktionen aus Beispiel 4 wird mit Benzol im molaren Verhältnis 1:10 alkyliert. Dazu wird die Reaktionsmischung in einen Autoklaven (300ml) verbracht, der mit einem Rührer und einem Katalysatorkorb versehen ist. In den Katalysatorkorb werden 25 Gew% bezogen auf die Masse des Olefins an Katalysator Zeolith Mordenith (MOR) gefüllt. Der Autoklav wird verschlossen und zweimal mit Stickstoff (N₂) gespült. Der Autoklav wird dann auf 200°C aufgeheizt. Man läßt die Reaktionsmischung 12h reagieren, kühlt dann ab, filtriert etwaige Katalysatorpartikel aus der Reaktionsmischung und analysiert die Reaktionsmischung mittels Gaschromatographie-Massenspektrometrie Kopplung.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 8

Die Umsetzung der Alkylbenzolmischung aus Beispiel 7 zum Alkylbenzolsulfonat erfolgt analog der Beschreibung in Beispiel 6.

### Beispiel 9

Eine Mischung der C₁₀-/ C₁₁-/C₁₂-Olefin-Fraktionen aus Beispiel 4 wird mit Benzol im molaren Verhältnis 1:2 alkyliert. Dazu wird die Reaktionsmischung in einen Autoklaven (300ml) verbracht, der mit einem Rührer und einem Katalysatorkorb versehen ist. In den Katalysatorkorb werden 5 Gew% bezogen auf die Masse des Olefins an Katalysator Zeolith ZSM-12 gefüllt. Der Autoklav wird verschlossen und zweimal mit Stickstoff (N₂) gespült. Der Autoklav wird dann auf 180°C aufgeheizt. Man läßt die Reaktionsmischung 12h reagieren, kühlt dann ab, filtriert etwaige Katalysatorpartikel aus der Reaktionsmischung und analysiert die Reaktionsmischung mittels Gaschromatographie-Massenspektrometrie Kopplung.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 10

Die Umsetzung der Alkylbenzolmischung aus Beispiel 9 zum Alkylbenzolsulfonat erfolgt analog der Beschreibung in Beispiel 6.

### Beispiel 11

Eine C₁₂-Olefin-Fraktion aus Beispiel 2 wird mit Benzol im molaren Verhältnis 1:4 alkyliert. Dazu wird die Reaktionsmischung in einen Autoklaven (300ml) verbracht, der mit einem Rührer und einem Katalysatorkorb versehen ist. In den Katalysatorkorb werden 10 Gew% bezogen auf die Masse des Olefins an Katalysator Zeolith Beta (BEA) gefüllt. Der Autoklav wird verschlossen und zweimal mit Stickstoff (N₂) gespült. Der Autoklav wird dann auf 180°C aufgeheizt. Man läßt die Reaktionsmischung 12h reagieren, kühlt dann ab, filtriert etwaige Katalysatorpartikel aus der Reaktionsmischung und analysiert die Reaktionsmischung mittels Gaschromatographie-Massenspektrometrie Kopplung.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 12

Die Umsetzung der Alkylbenzolmischung aus Beispiel 11 zum Alkylbenzolsulfonat erfolgt analog der Beschreibung in Beispiel 6.

### Beispiel 13

Eine Mischung der C₁₀-/ C₁₁-/C₁₂-Olefin-Fraktion aus Beispiel 4 wird mit Benzol im molaren Verhältnis 1:4 alkyliert. Dazu wird die Reaktionsmischung in einen Autoklaven (300ml) verbracht, der mit einem Rührer und einem Katalysatorkorb versehen ist. In den Katalysatorkorb werden 10 Gew% bezogen auf die Masse des Olefins an Katalysator Zeolith MCM-22 gefüllt. Der Autoklav wird verschlossen und zweimal mit Stickstoff (N₂) gespült. Der Autoklav wird dann auf 200°C aufgeheizt. Man läßt die Reaktionsmischung 12h reagieren, kühlt dann ab, filtriert etwaige Katalysatorpartikel aus der Reaktionsmischung und analysiert die Reaktionsmischung mittels Gaschromatographie-Massenspektrometrie Kopplung.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 14

In einen beheizten (120°C) 10 L Vierhalskolben wird mit einer Pumpe 1L/h Oleum (65%) in konzentrierte Schwefelsäure eingeleitet. Durch die Schwefelsäure werden über eine Fritte 1301/h trockene Luft geleitet, die das SO₃ ausstrippen. Der mit SO₃ angereicherte Luftstrom (ca. 4% SO₃) wird in einem 2m-langen Fallfilmreaktor, bei etwa 40-50°C (10-15°C Doppelmantel-Wasserkühlung) mit einer Alkylbenzolmischung aus Beispiel 13 in Kontakt gebracht und diese sulfoniert. Das molare Verhältnis von SO₃/Alkylbenzol beträgt 1,01:1. Die Reaktionszeit im Fallfilmreaktor beträgt etwa 10sec. Das Produkt wird in einen Nachreifungsbehälter gepumpt wo es etwa 4-8h verweilt. Danach wird die Sulfonsäure mit 0,4 Gew% Wasser stabilisiert und mit NaOH zum Alkylbenzolsulfonat neutralisiert.

### Beispiel 15

Eine C₁₂-Olefin-Fraktionen aus Beispiel 2 wird mit Benzol im molaren Verhältnis 1:10 alkyliert. Dazu wird die Reaktionsmischung in einen Vierhalskolben (2L) verbracht, der mit einem Rührer, einem Thermometer, einem Rückflußkühler mit einer Gasableitung und einem Tropftrichter versehen ist. Im Kolben werden Benzol und AlCl₃ vorgelegt, die Temperatur auf 80°C erhöht und langsam die Olefinmischung zudosiert. Man läßt die Reaktionsmischung ½ h nachreagieren, kühlt dann ab, filtriert etwaige Katalysatorpartikel aus der Reaktionsmischung und neutralsisiert die Reaktionsmischung mit NaOH. Danach wird mit Wasser gewaschen und das Produkt getrocknet.
Überschüßiges Benzol und Leichtsieder werden abdestilliert und das erhaltene Alkylarylgemisch mittels Gaschromatographie-Massenspektrometrie Kopplung und C¹³-NMR analysiert.

### Beispiel 16

Die Umsetzung der Alkylbenzolmischung aus Beispiel 15 zum Alkylbenzolsulfonat erfolgt analog der Beschreibung in Beispiel 6.

## Patentansprüche

1. Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens an einem Dimerisierungskatalysator zu einem C₁₀₋₁₂-Olefine enthaltenden Gemisch und gegebenenfalls Abtrennung der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen,
d) Sulfonierung der in Stufe c) erhaltenen alkylaromatischen Verbindungen und Neutralisation zu Alkylarylsulfonaten,

2. Verfahren nach Anspruch 1, **dadurch gekennzeichnet, daß** der Metathesekatalysator in Stufe a) ausgewählt ist aus Verbindungen eines Metalls der Gruppe VIb, VIIb der VIII. Nebengruppe des Periodensystems der Elemente.

3. Verfahren nach einem der Ansprüche 1 oder 2, **dadurch gekennzeichnet, daß** man in Stufe b) einen Dimerisierungskatalysator einsetzt, der wenigstens ein Element der VIII. Nebengruppe des Periodensystems der Elemente enthält, und man die Katalysatorzusammensetzung und die Reaktionsbedingungen so wählt daß ein Dimerengemisch erhalten wird, das weniger als 10 Gew.-% von Verbindungen enthält, die ein Strukturelement der Formel I (Vinylidengruppe) 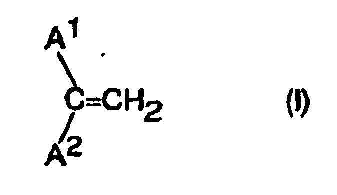 worin A¹ und A² aliphatische Kohlenwasserstoffreste sind, aufweisen.

4. Verfahren nach einem der Ansprüche 1 bis 3, **dadurch gekennzeichnet, daß** die in Stufe b) erhaltenen Olefine einen Anteil unverzweigter Olefine von unter 25 Gew.-% aufweisen.

5. Verfahren nach einem der Ansprüche 1 bis 4, **dadurch gekennzeichnet, daß** mindestens 80 % der in Stufe b) erhaltenen Olefine im Bereich von ¼ bis ¾, vorzugsweise von 1/3 bis 2/3 der Kettenlänge ihrer Hauptkette eine Verzweigung oder zwei Verzweigungen an benachbarten C-Atomen aufweisen.

6. Verfahren nach einem der Ansprüche 1 bis 5, **dadurch gekennzeichnet, daß** in Stufe c) ein Alkylierungskatalysator eingesetzt wird, der zu alkylaromatischen Verbindungen führt, die im Alkylrest 1 bis 3 Kohlenstoffatome mit einem H/C-Index von 1 aufweisen.

7. Alkylaryle als Zwischenprodukt, erhältlich in dem Verfahren gemäß einem der Ansprüche 1 bis 6.

8. Alkylarylsulfonate, erhältlich nach einem Verfahren gemäß einem der Ansprüche 1 bis 6.

9. Verwendung von Alkylarylsulfonaten gemäß Anspruch 8 als Tenside.

10. Verwendung nach Anspruch 9 in Wasch- und Reinigungsmitteln.

11. Wasch- und Reinigungsmittel, enthaltend neben üblichen Inhaltsstoffen Alkylarylsulfonate gemäß Anspruch 8.

## Claims

1. A process for the preparation of alkylarylsulfonates by
a) reaction of a C₄-olefin mixture over a metathesis catalyst for the preparation of an olefin mixture comprising 2-pentene and/or 3-hexene, and optional removal of 2-pentene and/or 3-hexene,
b) dimerization of the 2-pentene and/or 3-hexene obtained in stage a) over a dimerization catalyst to give a mixture comprising C₁₀₋₁₂-olefins, and optional removal of the C₁₀₋₁₂-olefins,
c) reaction of the C₁₀₋₁₂-olefin mixtures obtained in stage b) with an aromatic hydrocarbon in the presence of an alkylating catalyst to form alkylaromatic compounds,
d) sulfonation of the alkylaromatic compounds obtained in stage c), and neutralization to give alkylarylsulfonates.

2. The process according to claim 1, wherein the metathesis catalyst in stage a) is chosen from compounds of a metal of transition group VIb, VIIb or VIII of the Periodic Table of the Elements.

3. The process according to either of claims 1 or 2, wherein, in stage b), a dimerization catalyst is used which comprises at least one element from transition group VIII of the Periodic Table of the Elements, and the catalyst composition and the reaction conditions are chosen such that a dimer mixture is obtained which comprises less than 10% by weight of compounds which have a structural element of the formula I (vinylidene group) 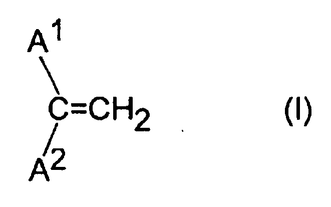 in which A¹ and A² are aliphatic hydrocarbon radicals.

4. The process process according to any of claims 1 to 3, wherein the olefins obtained in stage b) have a proportion of unbranched olefins of less than 25% by weight.

5. The process according to any of claims 1 to 4, wherein at least 80% of the olefins obtained in stage b) have one branch or two branches on adjacent carbon atoms in the range from 1/4 to 3/4, preferably from 1/3 to 2/3, of the chain length of their main chain.

6. The process according to any of claims 1 to 5, wherein, in stage c), an alkylation catalyst is used which leads to alkylaromatic compounds which have, in the alkyl radical, 1 to 3 carbon atoms with an H/C index of 1.

7. An alkylaryl as intermediate obtainable in the process according to any of claims 1 to 6.

8. An alkylarylsulfonate obtainable by a process according to any of claims 1 to 6.

9. The use of an alkylarylsulfonate according to claim 8 as surfactant.

10. The use according to claim 9 in detergents and cleaners.

11. A detergent or cleaner comprising, in addition to customary ingredients, an alkylarylsulfonate according to claim 8.

## Revendications

1. Procédé de fabrication de sulfonates d'alkylaryle selon les étapes suivantes :
a) on fait réagir un mélange d'oléfines en C₄ sur un catalyseur de métathèse pour fabriquer un mélange d'oléfines contenant du 2-pentène et/ou du 3-hexène et, éventuellement, on sépare le 2-pentène et/ou le 3-hexène,
b) on effectue la dimérisation du 2-pentène et/ou du 3-hexène obtenus à l'étape a) sur un catalyseur de dimérisation, ce qui permet d'obtenir un mélange contenant des oléfines en C₁₀₋₁₂ et, éventuellement, on sépare les oléfines en C₁₀₋₁₂,
c) on fait réagir les mélanges d'oléfines en C₁₀-C₁₂ obtenus à l'étape b) avec un hydrocarbure aromatique en présence d'un catalyseur d'alkylation pour former des composés alkylaromatiques,
d) on effectue la sulfonation des composés alkylaromatiques obtenus à l'étape c) et on neutralise pour obtenir des sulfonates d'alkylaryle.

2. Procédé selon la revendication 1, **caractérisé en ce que** le catalyseur de métathèse de l'étape a) est choisi parmi des composés d'un métal appartenant aux groupes VIb, VIIb du 8 ^{ème} groupe secondaire du système périodique des éléments.

3. Procédé selon l'une quelconque des revendications 1 ou 2, **caractérisé en ce que** l'on utilise, à l'étape b), un catalyseur de dimérisation, qui contient au moins un élément du 8^{ème} groupe secondaire du système périodique des éléments, et **en ce que** l'on choisit la composition catalytique et les conditions réactionnelles de manière à obtenir un mélange de dimères qui contienne moins de 10 % en poids de composés présentant un élément structurel de formule I (groupement vinylidène) : 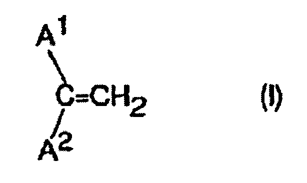 dans laquelle A¹ et A² sont des radicaux d'hydrocarbure aliphatique.

4. Procédé selon l'une quelconque des revendications 1 à 3, **caractérisé en ce que** les oléfines obtenues à l'étape b) présentent une fraction d'oléfines non ramifiées inférieure à 25 % en poids.

5. Procédé selon l'une quelconque des revendications 1 à 4, **caractérisé en ce que** 80 % au moins des oléfines obtenues à l'étape b), présentent une ou deux ramifications sur des atomes de carbone voisins, dans une plage de 1/4 à 3/4, de préférence, de 1/3 à 2/3 de la taille de leur chaîne principale.

6. Procédé selon l'une quelconque des revendications 1 à 5, **caractérisé en ce que** l'on utilise, à l'étape c), un catalyseur d'alkylation qui permet l'obtention de composés alkylaromatiques présentant 1 à 3 atomes de carbone dans le radical alkyle, avec un indice H/C de 1.

7. Alkylaryles comme produits intermédiaires, pouvant être obtenus par le procédé selon l'une quelconque des revendications 1 à 6.

8. Alkylarylsulfonates que l'on peut obtenir à l'aide d'un procédé selon l'une quelconque des revendications 1 à 6.

9. Utilisation d'alkylarylsulfonates selon la revendication 8, comme agents tensioactifs.

10. Utilisation selon la revendication 9, dans des produits de lavage et de nettoyage.

11. Produits de lavage et de nettoyage contenant, outre les substances habituelles, des alkylarylsulfonates selon la revendication 8.
